(19) Europäisches Patentamt
European Patent Office
Office européen des brevets

(11) **EP 4 108 770 A1**

(12) **EUROPEAN PATENT APPLICATION**

(43) Date of publication:
**28.12.2022 Bulletin 2022/52**

(21) Application number: **21181317.5**

(22) Date of filing: **24.06.2021**

(51) International Patent Classification (IPC):
**C12N 15/11** (2006.01)

(52) Cooperative Patent Classification (CPC):
**C12N 15/111;** C12N 2310/16; C12N 2320/13

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Aptamimetics GmbH**
**79252 Stegen (DE)**

(72) Inventors:
• **Castonguay, Jan Richard**
**79252 Stegen (DE)**

• **Siegert, Peter**
**79102 Freiburg im Breisgau (DE)**

(74) Representative: **Mertzlufft-Paufler, Cornelius et al**
**Maucher Jenkins**
**Patent- und Rechtsanwälte**
**Urachstraße 23**
**79102 Freiburg im Breisgau (DE)**

Remarks:
The complete document including Reference Table(s) and the Sequence Listing(s) can be downloaded from the EPO website

(54) **AGE-SELEX METHOD FOR APTAMER IDENTIFICATION AND USE**

(57)    The invention relates to a specific SELEX based method named AGE-SELEX which includes various specific steps including at least one bioactivity determination step allowing to obtain aptamers with high bioactivity modulation properties.

Fig. 2

**Description**

The invention relates to a method of identifying aptamers capable of eliciting modulation of biological functions (biochemical or biophysical reactions) in *in vitro systems,* such as isolated enzymes, components of signalling pathways, cell compartments or cell components, or in living biological systems (*in vivo*), such as cells, tissues and multicellular organisms, which allows to identify, select and obtain as well as to use, e.g., for research, diagnostic or therapeutic purposes, aptamers with good yield, high affinity and/or high specificity.

Background of the Invention

Aptamers are nucleic acid or nucleic acid based binding molecules in the form of short single-stranded DNA, RNA or synthetic XNA molecules, typically with about 10 to about 65 nucleotides, that can bind to and interact with targets of diverse nature due to their ability to fold into tertiary structures. They can, for example, specifically and with high affinity bind to various molecular entities, such as biomolecules, e.g., proteins including glycosylated, phosphorylated or otherwise modified proteins, polysaccharides, lipids or the like, and even small molecules and ions. Thus, they are similar to antibodies, but have properties that are advantageous in comparison to antibodies, for example shorter generation time (with no need for animals), low cost of manufacturing, good accessibility, avoidance of batch-to-batch variations, defined atomic composition (no glycosylation pattern, disulphide bonds etc.), higher accessibility to modification (e.g. by chemical synthesis - various chemical modification can be introduced at will in virtually any position of the aptamer molecule), high thermal stability (and thus, e.g., longer shelf life) and more targeting potential (even to non-immunogenic and/or toxic targets) due to their more versatile binding properties. Aptamers can be synthesized conveniently by chemical (de novo) synthesis, are chemically stable, have low immunogenicity, offer a robust platform to perform structural design and engineering and have small sizes in the range around about 25 Å and about 15 kDa. In contrast, antibodies require complex *in vivo* generation (e.g. requiring animals and antigen must show immunogenicity) and time-consuming and cost-intensive purification steps, as the target molecule is present in the raw product in a complex matrix. Moreover, antibodies are fragile as they are protein based and their tertiary structure is susceptible to changes (denaturation is irreversible), they can cause undesired immunogenic response, it is complex to modify them and they are rather large (around about 150 Å and about 150 kDa) and therefore less atom economic compared to aptamers.

Aptamers, due to their interaction and binding properties, have high potential and are partially already used in basic research (e.g., analytics), food safety, environmental monitoring, diagnostics and therapy. Applications in biosensing, biotechnology and biomedicine are made possible by their properties (see, e.g., Y. Zhang et al., Molecules 2019, 24, 941).

Various endeavours were undertaken in order to identify and generate aptamers. An especially important method includes the **S**ystematic **E**volution of **L**igands by **EX**ponential enrichment (SELEX) approach and numerous variations thereof.

The SELEX method is well established and is based on the manufacture of single strand random oligonucleotide derivative libraries where the individual oligonucleotides have about 40 to about 120 nucleotides, comprising a random region in the middle and fixed sequences (especially PCR primers) at the ends. The library is exposed to a target of interest, and any molecules not binding are removed, while the binding oligonucleotides are retained. Binders are then eluted and amplified using the Polymerase Chain Reaction (PCR), resulting in double-stranded DNA (dsDNA) oligonucleotides from which then single stranded oligonucleotides can be isolated. Several selection rounds can follow, leading to more specific and/or affine aptamers, and the resulting DNA oligonucleotides (aptamers) with high affinity and specificity are enriched in the selection pool and characterized by sequencing. After the sequencing, the identified aptamers can then be synthesized chemically in the amounts required for their use. Where RNA or RNA analogues are used as library, a reverse transcription into DNA is required before the PCR amplification together with a following in vitro transcription back into RNA. Various modifications of the SELEX procedure have been designed (see Y. Zhang *et al.,* loc. cit.). A specific variant of the SELEX procedure includes the use of cells having certain targets on their surface and allows to identify the binding of aptamers to such targets (see, e.g., Man Chen et al., Int. J. Mol. Sci 2016, 17(12), 2079). Among the weaknesses of this method, it may be mentioned that binding (and thus a one-dimensional characterization) is the only selection criterion, the quality of the readout depends on the cell status, the resolution is low due to usually unspecific elution, and the readout is limited to the yield of the isolated aptamer candidate pool.

Description of the Present Invention in General

The present invention, while also including steps that correspond to a normal SELEX procedure, contains various refinements and specific steps that, taken together, allows to isolate, from a library of synthesized aptamers, such specific molecules that have a specific bioactivity, e.g., an effect on biochemical or biophysical reactions, either *in vitro* or *in vivo,* including as examples (see further below) modulation of physiological functions, within isolates out of cells, cell compartments and especially cells, tissues or whole multicellular organisms. They thus allow to make available aptamers suitable for research, medical, pharmaceutical, diagnostic or physiologic purposes.

The main important steps included in the method and method steps of the present invention include a selection of aptamers by binding them to cells expressing targets of interest, determining of bioactivity and various further steps presented in detail below. Certain steps to individualize the discovered aptamers and to further develop new aptamers e.g., by including mutational PCR, the elution with target molecules or the inclusion of cells with a modified target to refine affinity and/or specificity of aptamers may also form important embodiments or components of the invention.

Most former improvements of the SELEX procedure focused on target preparation, e.g., presentation on solid phase or in solution, in aptamer modification (e.g., the base, other chemical modifications, extender, conjunctions), the PCR strategies to alter aptamers, the way of library generation (e.g., random, directed evolved, in silico simulated), or the method setup (e.g., manual vs semi-automated flow systems).

The present invention aimed at and resulted in improving the scope of analysis (not only binding), selection of the library (not as a whole but on single candidate sequence level) and allowing for cyclic evolution of the library.

What is most important, however, is that the method according to the present invention makes it possible to add a further characterization dimension to the SELEX and CELL SELEX methods, namely the dimension of bioactivity (modulation or evocation of a cellular signalling, biochemical or biophysical reaction).

Detailed Description of the Invention

The method of the invention, named AGE-SELEX (for **A**ctivity **G**uided **E**nhanced SELEX), in one embodiment, comprises two or more AGE-SELEX selection rounds including the following steps:

- In a first Step (1) - directly or after an additionally included preceding negative selection step as described below as Step (3*), with this preceding negative selection step here then referred to as Step (1*), using the resulting supernatant with an unbound oligonucleotide mixture - an oligonucleotide mixture (aptamer library), including DNA, RNA or XNA oligonucleotides comprising synthetic nucleotides preferably including terminal PCR primer sequences, is incubated with target carrying cells (preferably grown as monolayers) that display a target of interest or a modified target of interest, e.g., a truncated mutants, subdomain-depleted complexes or splice variant version, where alternating targets might be employed during different AGE-SELEX rounds allowing to evolve aptamers which only bind to specific regions of the target of interest and to further narrow down the aptamer binding site; this is followed by

- a Step (2) where the unbound oligonucleotides are removed by a washing step (and can be/are discarded);

- in a subsequent Step (3) (a recovery step for the bound aptamers), the target carrying cells with the bound oligonucleotides are treated with a (man-made, unnatural (e.g. chemically modified natural or a synthetic drug compound) or especially natural) ligand to a target of interest, so that the specifically bound nucleotides are set free and then recovered from the resulting eluate of the target materials, especially cell cultures or cell suspensions;

- optionally, in order to yet enhance the specificity of bound oligonucleotides to the target and to remove unspecifically bound oligonucleotides, a negative selection step is performed as an incubation Step (3*) with target negative cells (that is, cells different from the cells used in Step (2) not allowing for specific binding to the target of interest), especially cells inherently not exposing the target of interest, cells with altered targets, such as cells resulting from enzymatic inactivation or digestion of the target of interest or cells exposing a truncated or spliced target of interest, or in particular target knock-out cells follows, with subsequent removal of oligonucleotides that remain (unspecifically) bound on the cells,

- while the supernatant resulting from Step (3) or optional Step (3*) with unbound oligonucleotides (candidate aptamers) is used in the next step, where

- in a Step (4) forming part of the method according to the invention, the oligonucleotides are - if necessary (i) after Reverse Transcription (RT) into DNA starting from RNA or especially RNA analogues; or (ii) after (what we call here) modified Reverse Transcription (mRT) into DNA starting from DNA analogues (modified DNA) - amplified; where optionally Step (4) includes as Step (4*) an error-prone PCR in order to induce mutations, thus allowing a kind of aptamer evolution; and the concentration of the resulting oligonucleotide mixture is quantified before any subsequent Step (5), especially with digital droplet PCR; and

- the oligonucleotide mixture resulting from the amplification or the error-prone PCR is then, if necessary, adjusted to a desired concentration and subsequently subjected in at least one of the AGE-SELEX rounds passed according to the method of the invention, preferably in two or more or all of the rounds, to a bioactivity determination, including a series of steps, together with Step 4 forming a bioactivity loop, as follows:

○ in a Step (5) that is present in at least one of the rounds of the method but may be omitted in one or more rounds, the oligonucleotide mixture is subjected to a limiting dilution followed by amplification of (essentially) single molecular oligonucleotide fractions of sequences in order to obtain fractions with (essentially) only one, or very few different individual sequences; the fractions (partitions) with the resulting singled-out sequences are then partially saved while the rest, respectively, or where Step 5 is omitted (preferably aliquots of) the oligonucleotide mixtures of Step (4) or (4*), is or are subjected to

○ a Step (6), where

- (i) either, where RNA aptamers are desired, the DNA oligonucleotides are transcribed to yield the respective RNA oligonucleotides as RNA or especially RNA derivatives, e.g., 2'-modified RNA, or
- (ii) where DNA aptamers are desired, the DNA oligonucleotides are either treated by separation of the desired sense-strand from the antisense-strand which is discarded, to yield the respective single strand sense oligonucleotides as DNA or DNA derivatives or the amplification in Step (5) is performed as an asymmetrical PCR yielding an excess of the desired sense-strand; or a combination of both;

○ in a following Step (7) that is present in at least one of the rounds of the method but may be omitted in one or more other rounds - in such a case step 5 is omitted as well - , the bioactivity of the oligonucleotides in different partitions obtained in Step (6) is determined (especially the modulation of a biochemical or biophysical reaction, e.g. causing an inhibition of a function (antagonist aptamers), an activation of a function (agonist aptamers) or other, e.g., complex formation, modulation) by individual testing, optionally followed by a reverse transcription and saving of aliquots of aptamer-sequences in the respective partitions for later sequencing;

○ optionally, in a Step (8), in a bioactivity screening feedback loop, the oligonucleotide fractions found to be active are pooled and subjected to a further amplification Step (4), optionally including an error-prone PCR (as mentioned as Step (4*) above),
and with the resulting oligonucleotide mixtures of this Step (4) or (4*) either

- another Step (6) is performed followed by subjecting the resulting oligonucleotides to another sequence of Steps (1) / (1*) to (9), optionally including Step (8), said sequence of Steps constituting an AGE-SELEX round or
- another Step (6) is performed followed by subjecting the resulting oligonucleotides to another sequence of Steps (1) / (1*) to (9) where, however, Steps (5), (7) and (8) are omitted, thus leading to an oligonucleotide mixture pool (without individualized sequences), which bypasses the bioactivity determination, or
- another sequence of Steps (5) to (7), optionally including Step (8), is performed (additional bioactivity screening feedback loop);

○ in a Step (9), an oligonucleotide pool is formed, where the oligonucleotide pool consists of either

- oligonucleotide fractions derived by a sequence of Steps (1) / (1*) to (7), optionally Step (8), where the oligonucleotides are selected based on their bioactivity performance in Step (7) and picked from the saved fractions (partitions) from Step (5), or
- oligonucleotide fractions resulting from an AGE-SELEX round where the bioactivity determination is skipped (bypassed) in that only the Steps (1) / (1*) to (6), omitting Step (5) (limiting dilution), are performed;

○ the resulting pool is then either

- recycled into another AGE-SELEX round as described above (Steps (1) / (1*) to (9), optionally including Steps (5) and (7) and/or (8)) or
- into an AGE-SELEX round (Steps (1) / (1*) to (9), optionally including Step (5) and (7) and/or (8)), where the oligonucleotides are incubated (in a step analogous to Step (1)) with cells comprising a modified target, e.g., a truncated or spliced variant of a target or specific parts thereof that are thought to be or are of interest to be examined for being important for a biologic activity;

with the proviso that Steps (1), (2), (3), (4), (6) and (9) are obligatory in each round and both of Steps (5) and (7) are present in at least one round and/or preferably in the last round. Depending on the quality of the results of Step (7), Step (8) may be performed.

Finally, in an optional concluding Step (10) the aptamer sequencing pool is generated. This pool includes the oligonucleotide pool derived in Step (9) in the last AGE-SELEX round and optionally oligonucleotide sequences saved in previous AGE-SELEX rounds. The sequencing pool, thus containing only aptamers found and isolated using the above-mentioned AGE-SELEX method, is sequenced (e.g. as described in the Examples section) and, where desired, optionally chemical analogues of the aptamers thus found may be synthesized in order to achieve aptamers with desired chemical properties, e.g., convenient way of manufacture, resistance to nucleases and/or cell uptake/binding.

This method (the method of the invention), which is also referred to as AGE-SELEX herein (for Activity Guided Enhanced SELEX), offers various improvements over prior art methods to characterize the binding of aptamers: Both, binding <u>and</u> bioactivity are characterized and used as criteria for selection of aptamers. The method makes it possible to analyse, identify and obtain aptamers on a single molecule level (high method resolution). There is a highly reduced rate of "false positive" in terms of bioactivity of the obtained aptamers. The method allows for intrinsic tight library tuning (e.g., due to the possibility to include target variants and mutational PCR). And the method can be automated in order to achieve high throughput screening.

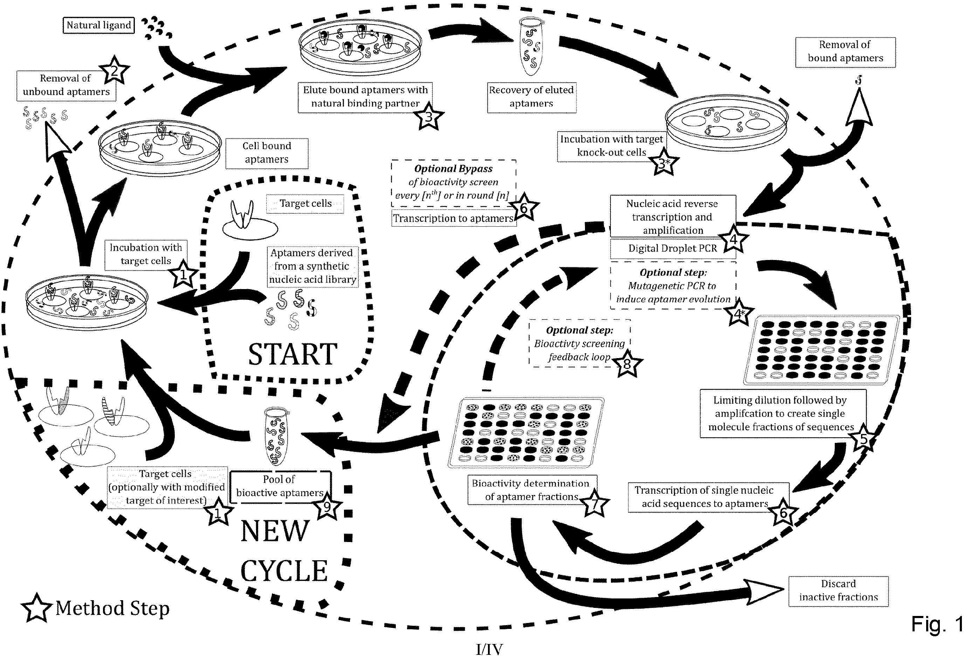

Referring to Fig. 1, which is included here for illustration and visualization purposes without limiting the scope of the invention, though showing a representation of a complete round of the AGE-SELEX process including obligatory and optional steps according to the invention, "Incubation with target cells" corresponds to Step (1), "Removal of unbound aptamers" corresponds to Step (2), "Elute bound aptamers with natural binding partners" and "Recovery of eluted aptamers" corresponds to Step (3), optional "Incubation with target knock-out cells" and "Removal of bound aptamers corresponds to optional Step (3*), "Nucleic acid amplification" corresponds to Step (4), "Limiting dilution followed by amplification to create single molecule fractions of sequences" corresponds to Step (5), "Transcription of single nucleic acid sequences to aptamers" corresponds to Step (6) (i), "Bioactivity determination of aptamer fractions" corresponds to Step (7), "Optional step: Bioactivity screening feedback loop" corresponds to Step (8), "Pool of bioactive aptamers" corresponds to Step (9) and "Incubation with target knock-out cells" corresponds to Step (3*), "Target cells with modified target of interest" corresponds to a variant of Step (1). This explanation is thought to help get an overview of the method of the invention, but is not thought to be limiting it; although the Figure also refers to a preferred embodiment of the invention. Note the numbers in the stars in Fig. 1 correspond to the Step number (Step 1, ...) of the AGE-SELEX method described herein.

The method of the invention preferably includes more than one, for example 2 to 20, especially 5 to 15, in particular 8 to 12, e.g. 10, rounds from Step (1) up to Step (9), or variants thereof including or omitting Steps mentioned to be "optional", and then the final Step (10). With regard to Step (10), intermediate sampling of aliquots for sequencing steps after the amplification in Step (4) or (5) is possible.

A "round" (or "cycle") is to be understood as any sequence including Step (1) (optionally with preceding Step (1*), Step (2), Step (3) optionally including Step (3*), Step (4), optionally including Step (4*), optional Step (5), Step (6), optional Step (7), optional feedback loop Step (8), and Step (9), that is, all Steps actually involved in one AGE-SELEX round from Step (1) or (1*) to Step (9).

The treatment in Step (3) comprising elution of bound aptamers with a natural binding partner for the target of interest allows to greatly reduce the number of false positive candidate aptamers (namely aptamers with strong binding properties but no or low biological activity) and to limit the candidate pool based on the binding site on the target.

The treatment in Step (3*) (also corresponding to Step (1*)) allows for a further selection by allowing to eliminate (at least part of) oligonucleotides not binding to the target of interest, thus diminishing the number of remaining aptamer candidate oligonucleotides to be further selected.

The treatment in Step (8) comprising error-prone (and thus especially mutagenic) PCR allows to alter the candidate pool of aptamers, leading to a directed evolution by selection of the most affine and/or selective aptamers for the bioactivity of interest.

The limiting dilution/ single nucleic acid amplification Step (5) as well as Step (6) (i) or (ii) allow to split the candidate pool into fractions comprising single (or very few, e.g., 1 to 3) sequences, to create in-cycle backup copies of candidate sequences for monitoring the library over two or more cycles ("rounds") of all steps; and allows to generate homogenous aptamer panels of candidate sequences. Step (5) alone or in combination with any one of Steps (5) and Step (6) (i) or Step (6) (ii), in addition to being present in the AGE-SELEX method according to the invention, form independent embodiments of the invention as part of any SELEX process, as they are not used in any SELEX process known so far and allow to achieve isolated nucleotides or very low (e.g. about 1 to about 3) numbers of sequences per fraction with identical (or if more than 1, e.g. about 3, sequences with appropriate bioactivity) sequence per fraction each.

A special embodiment of the invention thus relates to a SELEX process including Step (5) alone or in combination with any one of Steps (6) (i) and (6) (ii). In this embodiment, an oligonucleotide mixture is subjected to a limiting dilution followed by amplification of (essentially) single molecular oligonucleotide fractions of sequences in order to obtain fractions with (essentially) only one, or very few different individual sequences; the fractions (partitions) with the resulting singled-out sequences can then be partially saved while the rest of the oligonucleotide is subjected to a next step in a customary SELEX cycle. Step (5) or Step (5) plus any one of Steps (6) (i) and (6) (ii) can preferably be executed as described herein-above or herein-below.

One of the central aspects of the method according to the invention is the bioactivity determination which allows to assess the bioactivity of thus interesting aptamer fractions - only aptamers with a desired bioactivity level can be selected in rounds where the bioactivity loop is made use of, and then used for the next cycle (starting with Step (1) or (1*)).

The optional bioactivity feedback loop (Step (8)) allows to alter the bioactivity by allowing subjecting found candidate sequences to an optional error-prone (thus as result mutagenic) PCR and to repeat the bioactivity screening cycle or directly continue with a new AGE-SELEX round with a resulted new subset of candidate oligonucleotides.

Step (1*) or Step (3*) with use of cells with altered binding targets allows to further increase method stringency by altering the binding target, e.g., by subdomain deletion or splice variants of a receptor target or the like, and to further narrow down the number of available binding sites to aptamers in order to obtain aptamers with higher binding specificity.

One own invention embodiment is formed by the bioactivity determination comprising the sequence of Steps (4) to (7) and, if used, Step (8) (the bioactivity loop). This is highly advantageous in that it allows to create single (or closely related) sequence aptamer fractions that show high binding and high bioactivity. It is an embodiment of central importance for the whole method according to the invention. To further sharpen the selecting effect of this loop, it is also possible to directly repeat, with the resulting mixture of a Step (4) after a preceding bioactivity loop, the sequence of Steps (5) to (7), resulting in a bioactivity loop directly followed by another bioactivity loop.

Another invention embodiment relates to Step (1), (1*) and (3*) which allows to further limit the binding of aptamers to the desired position in a certain target.

Another invention embodiment relates to the limiting dilution/amplification Step (5).

The invention, in a further embodiment, allows for the use of an aptamer found by the AGE-SELEX method in diagnostics, therapy or research and the aptamer found for use in diagnostic or therapeutic treatment, e.g. of warm-blooded animals, such as humans.

Another embodiment of the invention relates to the use of an aptamer found by the AGE-SELEX method, or for said aptamer for use, in the diagnosis; or in the treatment of a condition, disorder or disease in a warm-blooded animal, especially a human, especially in need of such treatment, or a corresponding method of diagnosis or especially treatment of said condition, disorder or disease, in the latter case comprising administering an aptamer found by the AGE-SELEX method to said warm-blooded animal, especially a human, preferably in an effective amount. Variants of this embodiment relate to the use of an aptamer found by the AGE-SELEX method for the manufacture of a medication for use in the mentioned treatment or of a diagnostic for use in the diagnosis of said condition, or a pharmaceutical preparation for use in said treatments.

The following definitions serve to define preferred meanings of more general expressions and features used herein, where in each invention embodiment one, more than one or all general expressions or features can be replaced by the more specific expressions or features, thus forming and disclosing specific embodiments of the invention, each of which is also to be regarded as included here as invention embodiment.

"Moreover" means that features characterized by this word may be of lower preference than the other features without this attribute.

"Comprising", "including", "with" or "containing" means that the features (which includes the term components) or list of features attributed with these words or other grammatical forms thereof are non-limiting features (other features may be present). "Consisting of" or "consist(s) of" refers to a closed (conclusive, exhaustive) list of features and excludes the presence of other features. In preferred variants of the non-limiting feature lists, "comprising", "including", "with" or "containing", or their grammatical analogues, can be replaced with "consisting of" or "consist(s) of".

Where "about" is mentioned, this preferably means that the numeric value to which this is added can vary by $\pm$ 20 %, more preferably by $\pm$ 10 %, yet more preferably by $\pm$ 5 %, and most preferably "about" where used can be deleted.

"Aptamers" are nucleic acids (DNA, RNA) or nucleic acid derivatives (XNA), also referred herein to as "oligonucleotides", especially in the description of AGE-SELEX rounds, where the designation "aptamer" or "candidate nucleotide" or "aptamer candidate" may be used at any stage, however, preferably, the oligonucleotides after the final AGE-SELEX round are named aptamers, as they (in addition to having a secondary structure typical for aptamers, such as hairpins, loops, single stranded and double stranded segments) show the preferred binding properties desired according to the present invention, especially RNA or preferably chemically (especially 2'-, e.g., 2'-fluoro or 2'-methoxy) modified DNA or RNA, often with about 8 to 100, preferably 10 to 65 nucleic acid (if given derivative) units, that bind selectively and with distinct affinity to targets or target parts (similar to epitopes in the case of antibody binding). While the corresponding libraries (which are actually oligonucleotide (derivative) libraries) and aptamers defined below also comprise (in the case of the libraries presumably even mainly) molecules that are not binding to the target of interest, they are nevertheless also referred to herein as aptamer libraries and aptamers, respectively. In the stricter sense, aptamers are nucleic acids or nucleic acid derivatives that bind to a target of interest, that is, the positive result of the method according to the invention. They can then be called "true aptamers" herein.

Usually, modified DNA or RNA molecules (DNA or RNA derivatives) are preferred as this allows to avoid the susceptibility to nucleases, e.g., the virtually omnipresent RNases. Among the modifications (also referred to herein where nucleotide or nucleic acid analogues are mentioned), Spiegelmers (i.e. with L-nucleotides), SOMAmers, locked nucleic acids (LNA), bridged nucleic acids (BNA), RNA with modified base pairs or nucleobases, such as 7-(2-thienyl) imidazo [4, 5-β] pyridine (Ds), 2-nitro-4-propynylpyrrole (Px), 5SICS-MMO2, 5SICS-NaM, 5SICS-DMO, isoG-5-Me-isoC, isoC-isoG, (enol)isoG-thymine or AF-Z, S-Pa, base pairs, RNA comprising artificial nucleotides, e.g. 2-amino-8-(10-β-D-2-deoxyribofuranosyl)-imidazol[1,2-a]-1,3,5-triazin-4(8H)-one (P) or 6-amino-5-nitro-3-(10-β-D-20-deoxyribofur-anosyl)-2(1H)-pyridone (Z), RNA with modified bases, such as 5-methyluridine, pseudouridine, dihydrouridine or C5-ethynyl-deoxyuridine or its reaction product with azide (preferably bound to a carrier material for synthesis of the aptamers), backbone modified RNAs, such as morpholino, peptide nucleic acids, phosphorothioate, boranophosphate variants, sugar modified nucleotide comprising RNAs, such as cyclohexenyl, altritol, arabinose, 2'-fluoroarabinose, 2'-fluoroarabino nucleic acid, preferably 2'-O-methyl ribonucleotide or especially 2'-fluoro ribonucleotide comprising RNA (which can be synthesized using RNA polymerases or reverse transcriptase) are to be mentioned, without limiting the scope of possible modifications. Examples for such modifications and references for their synthesis can be found in T. Wang et al., loc. cit. Biotechnology Advances 37 (2019), 28-50 and A. S. Thompson et al., Biochemistry 2020 Aug 11;59(31):2833-2841. 2'-modified (e.g., 2'-fluoro or 2'-methoxy) RNA or DNA is preferred, especially as it can also be manufactured using polymerases.

The optional chemical synthesis of aptamers allows to synthesise also RNA or DNA derivatives including chemical modifications by (e.g. 2'-) modified oligonucleotides that are not accessible to nucleases.

Aptamer libraries (actually more generally oligonucleotide libraries), as they are used as starting materials in the methods according to the invention, can be prepared according to well-known manufacturing methods as random sequence pools or applying selective preferences of possible resulting library members (e.g. by choice of the educts or their pairing properties, e.g. using pre-formed oligomers (short prefabricated segments with 2 or more nucleotides) as starting materials, or by forming patterned aptamer libraries). They may be of the DNA or preferably of the RNA or yet more preferably the XNA type, the latter encompassing one or more chemical modifications compared with the natural DNA or RNA components, e.g., nucleic acid analogues, and thus having more hydrolysis resistance than their natural counterparts. The aptamer molecules in the library usually comprise a more or less randomized region, flanked by two fixed primer-binding sequences on the two ends of each molecule in order to enable amplification of the (later enriched) sequences by PCR. Exemplary libraries, their manufacture and their properties are described, e.g., in T. Wang et al., loc. cit. The libraries preferably consist of single stranded oligos with a variable region flanked by two constant regions necessary for primer binding. The variable region typically has a length of 8 to 100, preferably 10 to 65, especially 25 to 60 bases (or nucleic acid (derivative) units). All or parts of the nucleic acids within the variable region consist of modified nucleic acids, e.g., 2'-methoxy- or preferably 2'-fluoro modified RNA, provided the employed Polymerases are able to accept the corresponding substrate.

Ligands are (preferably natural) compounds, e.g., growth factors, hormones, neuronal transmitters, cytokines, chemokines, or the like. They may belong to chemical classes like amines, carbohydrates, lipids, peptides, proteins, glycoproteins, lipoproteins, phosphoproteins, and the like. Preferably they are effectors, that is, they modulate a bioactivity, e.g., by agonizing or antagonizing (inhibiting) a biological activity.

In the first Step (1) of the method according to the invention, aptamer mixtures (aptamer libraries) with synthetic nucleotides (specifically nucleotide analogues) are incubated with target of interest, especially target cells (preferably grown as monolayers, or as cell suspensions) of interest that express a target of interest, e.g., in each case wild type (wt) or genetically engineered, yeast, bacterial (including cyanobacterial), or plant cells (e.g. algal), or preferably cells from multicellular animal organisms, especially warm-blooded animals, most especially humans, e.g. cells in blood, interstitial fluids, tissues or organs.

The term "target of interest" relates to any biological target (preferably expressed on biological target materials, in particular selected from cells, outer membranes of cells, preparations of the outer cell membrane or compartments in cells that communicate with the cell membrane (e.g., vesicles for internalization or secretion or externalization (e.g. Arc capsids, endocytosis vesicles or exocytosis vesicles or Golgi vesicles) carrying the target molecules, e.g., displayed on the outer cell membrane of a cell, moreover on membrane preparations of the cell membrane or compartment membranes in cells) the biological activity of which is to be examined or used, especially activated or deactivated or otherwise modulated, e.g., for research, diagnostic or therapeutic purposes. Such targets (of interest) preferably include but are not limited to the following: bacterial pili, phage or virus binding protein (including glycoprotein) molecules expressed on the cells, such as ACE-2 (e.g. in the case of SARS-CoV-2 spike protein) or CD4 molecules (e.g. in the case of gp120 of HIV and/or its chemokine co-receptors); enzymes (e.g. lipase or protease secreting cells or especially cell membrane bound enzymes); ligand binding proteins in signal-transduction pathways, biomolecules involved in modulation of generation of second messengers, such as lipid messengers, nitric oxide, superoxide, hydrogen peroxide, carbon monoxide or hydrogen sulfides, biomolecules involved in modulation of generation or secretion of signal molecules in inflammation processes, intracellular signal or signal cascade and of proteins and molecules associated therewith, e.g. protein kinases, especially serine/threonine, histidine or especially tyrosine kinases, extracellular receptors, e.g., G-protein-coupled receptors, Toll-like receptor activation and propagation of their effect, biomolecules involved in modulation of gene activation, such as activation or deactivation of any one or more of promotors, transcription, and translation, especially by ligand-mediated modulation of extracellular receptors, e.g. activation of sre-promoter leading to the expression of or when artificially coupled to reporter sequences, e.g, Green Fluorescent Protein, leading to their expression) (very preferred), extracellular or intracellular receptors or the like; such as especially reaction to growth factors (a preferred variant in all invention embodiments), e.g. EGF, based on binding of corresponding receptors present in the outer cell membrane, such as EGFR; biomolecules modulating cell-cell communication or interaction and molecules enabling it, such as hormones, tight junction proteins, modulation of proteins implied in adherens junctions or desmosomes, such as E-, N- and P-cadherins, desmoglein or desmocollin, gap junction proteins such as connexins; biomolecules involved in modulation of cell proliferation or cell division controlling proteins, apoptosis related proteins, such as TNFR1 or TNFR2, or Fas receptor (aka Apo-1 or CD95) or other caspase activators; molecules for the formation of cell walls; transmembrane proteins capable of modulation of electric membrane potential ,of ionic composition in the intracellular or especially extracellular medium (e.g. channels, such as leakage channels, ligand-gated channels, voltage-dependent channels, or ion pumps),
modulation of pH-regulation associated proteins, such as proton pumps, membrane bound transport proteins, e.g. ligand gated ion or proton channels, biomolecules involved in modulation of intracellular Calcium concentration; biomolecules involved in changes in metabolism (e.g. inside the cell or modulation of transport proteins leading to release or consumption or exposure of organic molecules such as sugars, amino acids, lipids, peptides or nucleotides, biomolecules involved in modulation or expression or function of transmembrane proteins not yet mentioned; binding of antibodies or antigen binding receptor proteins specific for a membrane-bound component of cells (e.g., MHC I or II or antigen receptors on leukocytes, e.g. lymphocytes); or the like.

In Step (1), the incubation (intended for binding of the "true" aptamers) preferably takes place after diluting the aptamer library to be tested in a binding buffer at elevated temperatures, especially from 30 to 99 °C, e.g., from 50 to 96 °C, followed by chilling, e.g. on ice. The biological targets with the "target of interest", especially cells, e.g. tumor cell line cells, such as wild type (wt) Caco-2 cells, are suspended or (especially in the case of cells) seeded on an appropriate medium (e.g. gelatin, agar or other polysaccharides) in appropriate vessels, especially cell culture dishes, and incubated with the aptamer library in a cell medium (e.g. forming a binding buffer), preferably at temperatures in the range from 4 to 40 °C, e.g. at 37 °C, for a time sufficient to allow the binding, e.g. about 5 to 60 minutes, e.g. 15 to 30 min.

This binding Step (1) is followed by a washing Step (Step (2)) with a washing buffer in order to remove weakly bound and unbound aptamers.

The removal of the unbound nucleic acids in Step (2) can be conducted by any method known in the art that allows to separate unbound molecules from cells, e.g., by washing once or more often, e.g., 2 to 5 times, with a washing buffer which removes weakly bound and unbound oligonucleotides. The supernatant from the washing step with the unbound aptamers is removed and can be discarded, while the aptamers bound to the material of interest remain bound.

In Step (3), which serves to set the bound aptamers free, the binding of a (preferably natural, but alternatively feasibly also an analogue thereof with similar binding characteristics, especially of a competitive kind) ligand (e.g. a growth factor, such as EGF) to a target of interest, e.g., a growth factor receptor, such as EGFR, preferably takes place under customary conditions, by incubating the cells or moreover other materials bearing the target of interest, the ligand or moreover ligand analogue in a solution, e.g. a ligand buffer solution, or suspension (e.g. in ligand buffer) comprising the material of interest, especially cells so that the specifically bound aptamers are set free and then recovered from the resulting eluate of the cell cultures or suspensions. The ligand or its form capable of specific binding is then removed from the resulting eluate solution of eluted (candidate) aptamers, e.g., by a chromatographic step (e.g., size exclusion chromatography, affinity chromatography) or by inactivation, e.g. by heating to, for example, 50 to 100 °C, preferably 70 to 98 °C, such as 95 °C (this heating is especially viable for peptide or protein ligands (such as growth factors, e.g. EGF) that are denatured due to the heating), or precipitation, e.g. with a solvent, resulting in a solution containing the selected aptamers.

In the optional Step (3*) or (1*), in order to yet enhance the specificity of bound nucleotides to the target and to remove unspecifically or weekly specifically bound aptamers, a negative selection step with a material not displaying the target of interest, especially a cell not expressing the target of interest, is included, e.g. with materials from cells not expressing the target of interest, e.g. due to molecular inhibition of the biosynthesis of the target of interest, especially an incubation with target knock-out cells, which cells can, e.g., be obtainable or obtained with the help of a gene or genome edition method, e.g. using mutagenesis using zinc finger nucleases, using the TALEN method or especially using a CRISPR method (for appropriate protocols, see e.g. M. Tofazzal Islam et al., eds., "Crispr-Cas Methods", Springer Protocol Handbooks, 2020), especially the CRISPR/Cas9 method, e.g. using commercial kits, such as the Origin KN214877 Human Gene Knockout Kit or any other appropriate method. In a preferred embodiment of Step (3*), the resulting knock-out cells are suspended in a medium otherwise allowing the binding of aptamers to the target of interest, or preferably seeded on plates and grown in a cell medium as just mentioned. The binding procedure is accomplished as described for Step (1), so that the aptamers binding unspecifically to the material or especially cells remain on the cells or cellular materials, while the supernatant including the not unspecifically bound aptamers is used further. Preferably, the resulting cells or cell materials not expressing the target of interest are incubated here, e.g., in a solution or after seeding on culture dishes with growing especially to confluency, with the aptamers (candidate sequences) from the preceding step (which may be chilled, e.g. on ice, before use), preferably at temperatures from 4 to 40 °C, such as at 37 °C, for an appropriate time, e.g. 10 to 60 minutes, such as 15 to 30 minutes, e.g. 20 min; followed by removal of the supernatant with the unbound aptamers (which are binding candidates - the unspecifically bound aptamers remain on the knock-out cells or cell parts).

Optional Step (3*) or (1*) may be included in the AGE-SELEX method according to the invention, preferably in at least one of the rounds, such as in two or three or four rounds, thus leading to further enhancement of selection of specific aptamers, for example as the selection pressure is increased in terms of specificity and binding strength leading to more specific and strong-binding aptamers.

As part of any of Steps (3) or (3*), the resulting supernatant containing ligand is treated to remove the ligand, e.g., by chromatography, or inactivate the ligand, e.g., by heating to, for example, 50 to 100 °C, preferably 70 to 98 °C, such as 95 °C.

The ligand free or inactivated ligand buffer solution containing the eluted aptamer fraction (the sample resulting from preceding Step (3) or (3*) is preferably further treated and recovered, e.g., by centrifuging or other treatment to remove particular remnants or cells, purification, e.g., with magnetic silica beads known to be useful to recover nucleic acids, such as the MagMAX Cell-Free Total Nucleic Acid Isolation Kit from Thermo Scientific, followed by elution of the aptamers in an elution buffer, for example, in a final volume of 2 to 50, e.g. about 15 μl.

The term bioactivity determination, or the determination or determining of bioactivity, or "bioactivity is determined", according to Step (7) may include any method of measuring biochemical (e.g. metabolic or enzymatic) or biophysical (e.g. absorption change or emission change for photons, electric potential across biological membranes) reaction, especially an activity (especially an agonizing, antagonizing or otherwise modulating) of a target of interest on a biological target material, e.g., DNA transfer by bacterial pili, inhibition of entry of bacteriophages or virus into cells by competitive or other binding to the phage or virus binding protein (including glycoprotein) molecules expressed on the cells, such as ACE-2 (e.g., in the case of SARS-CoV-2 spike protein) or CD4 molecules (e.g., in the case of gp120 of HIV and/or its chemokine co-receptors); modulation of enzyme function (e.g., of lipase or protease secreting cells or especially cell membrane bound enzymes); modulation of ligand binding proteins, modulation of signal-transduction pathways, modulation of generation of second messengers, such as lipid messengers, nitric oxide, superoxide, hydrogen peroxide, carbon monoxide or hydrogen sulfides, modulation of generation or secretion of signal molecules in inflammation processes, initiation of an intracellular signal or signal cascade and of proteins and molecules associated therewith, e.g., phosphorylation or dephosphorylation of protein kinases, especially serine/threonine, histidine or especially tyrosine kinases, modulation of activity of other extracellular receptors, e.g., G-protein-coupled receptors, Toll-like receptor activation and propagation of their effect, modulation of gene activation, such as activation or deactivation of any one or more of promotors, transcription, and translation, especially by ligand-mediated modulation of extracellular receptors, e.g. activation of sre-promoter leading to the expression of c-fos or when artificially coupled to reporter sequences, e.g, Green Fluorescent Protein,leading to its expression) and the measurement of its enzymatic activity (e.g. its enzymatic reaction leasing to fluorescence), intracellular receptor modulation, especially caused by interactions with membrane bound proteins that elicits it, or the like; such as especially reaction to growth factors, e.g. EGF, based on binding of corresponding receptors present in the outer cell membrane, such as EGFR; modulating cell-cell communication or interaction and molecules enabling it, such as hormones, tight junction proteins, modulation of proteins implied in adherens junctions or desmosomes, such as E-, N- and P-cadherins, desmoglein or desmocollin, gap junction proteins such as connexins; modulation of signal-transduction pathways, modulation of generation of second messengers, such as lipid messengers, nitric oxide, superoxide, hydrogen peroxide, carbon monoxide or hydrogen sulfides, modulation of generation or secretion of signal molecules in inflammation processes; modulation of cell proliferation or cell division controlling proteins, activity of apoptosis related proteins, such as TNFR1 or TNFR2, or Fas receptor (aka Apo-1 or CD95) or other caspase activators; modulation of molecules for the formation of cell walls; modulation of electric membrane potential by modulation of corresponding transmembrane proteins, modulation of ionic composition in the intracellular or especially extracellular medium by modulation of corresponding transmembrane proteins (e.g. channels, such as leakage channels, ligand-gated channels, voltage-dependent channels, or ion pumps), modulation of pH-regulation associated proteins, such as proton pumps, other modulation membrane bound transport proteins, e.g. ligand gated ion or proton channels, modulation of intracellular Calcium concentration; changes in metabolism (e.g. inside the cell or modulation of transport proteins leading to release or consumption or exposure of organic molecules such as sugars, amino acids, lipids, peptides or nucleotides, modulation of expression of function of transmembrane proteins not yet mentioned; modulation of binding of antibodies or antigen binding receptor proteins specific for a membrane-bound component of cells (e.g., MHC I or II or antigen receptors on leukocytes, e.g. lymphocytes); each of them either in isolated (especially outer) membranes of cells, compartments of cells (preferably as far as they interact with the medium outside cells, e.g. in endocytosis or exocytosis or Golgi vesicles), all preferably on intact cells in suspension or other cell culture, e.g. on dishes, and preferably relating to binding sites exposed on the outer cell membranes (or in case of fungal or plant cells or bacteria also on their cell walls), like especially extracellular receptors (receptor proteins), such as Growth factor receptors, in particular EGFR (these corresponding to bioactivity determination in the embodiments of the invention). For example, an eluted nucleotide fraction is centrifuged, purified with magnetic silica beads (e.g. with the MagMAC Cell-Free Total Nucleic Acid Isolation Kit from Thermo Scientific) and eluted in an elution buffer. All variants mentioned above relating to modulation by binding to components expressed in the cell membrane and exposing binding places for aptamers on the outside of the cell but still bound thereto are preferred. Where features overlap in the above enumeration, they form strict subsets which do not remove clarity as the non-overlapping parts and the overlapping parts do not change the total scope.

Among the methods of identification of such reactions, while no specific method known to the person skilled in the art is to be excluded, as specific examples there are to be mentioned: the activation by interacting aptamers of promoters leading to gene expression, especially to expression of genes or reporter constructs that subsequently lead to induction or diminution of fluorescence, phosphorescence or luminescence, by binding aptamers; induced inhibition of gene expression by aptamers, e.g. by quantitative real-time PCR; changes in the phosphorylation, methylation or acetylation state of the cell or respective proteins, e.g. by cell-based ELISA; induced protein-interaction, protein-binding or complex-formation, e.g. detected by western-blotting, circular dichroism spectroscopy or plasmon resonance spectroscopy; the change in cell volume; in case of secreted molecules analysis of the medium around cells or the cytosol or liquid from intracellular compartments, such as mitochondria or chloroplasts, e.g. using chromatographic methods, (e.g. HPLC, GC), spectroscopic methods, such as mass spectrometry, absorption or emission of electromagnetic radiation, e.g. light, UV or IR radiation, HPLC-NMR or the like, the examination of uptake or release of fluorescence or radioactively labelled molecules, electrophysiological methods, e.g. for determining the electric transmembrane potential, ion or proton measurement methods, e.g. determining the ionic composition, the pH measuring the viability of cells (e.g. using trypan blue exclusion), the measurement of ions by their quenching effect on fluorescence or bioluminescence, e.g. using fluorescent reporters, such as Fura-2 or genetically engineered variants of Green Fluorescent protein such as "Cameleon"; ion or pH specific sensors, cytometry, e.g. FACS, cell growth or proliferation measurement or other well-known methods and the corresponding well-known apparatus and equipment can be used.

In Step (4), the optional reverse transcription (RT) - (i), in case of RNA or RNA derivative nucleotides - or the optional modified Reverse Transcription (mRT) - (ii), in case of modified DNA - the mandatory PCR amplification is performed in a two-step reaction. In a specifically preferred variant with 2' modified RNA oligonucleotides, first, a reverse transcription step is performed according to Burmeister *et al.*, 2005, loc. cit., with an appropriate reverse transcription polymerase, e.g., with the Superscript IV Reverse Transcriptase (Invitrogen). Especially, in the case of RNA aptamers, the obtained PCR pool is amplified with a primer comprising a promoter for a RNA polymerase, especially a T7 promoter, and *in vitro* transcribed as described in the Examples section with the RGVG M6 polymerase according to Meyer *et al.* 2015, or other RNA polymerases known to the person skilled in the art.

After a buffer exchange, e.g., using Size Exclusion Spin Columns, such as Sephadex G-25 spin columns, the larger molecules (e.g., the oligonucleotides/aptamer analogues) pass the column, while smaller molecules, such as salts and mononucleotides, are retained on the column material. Alternatively, the RT and PCR steps are conducted without buffer exchange if possible (as can be decided on knowledge basis by the person skilled in the art).

The eluate is then subjected to a PCR amplification with an DNA polymerase, e.g. Q5 as specified in the Examples section, employing primers including a promoter (flanking) region, e.g. the T7 promoter flanking region, and its counterpart on the other DNA strand, the corresponding primer pair named T7 + RT-1 hereinafter, respectively) according to standard PCR conditions, e.g. as mentioned in the Examples Section, and thereafter subjecting the PCR product to a purification, preferably again spin-column based by binding of the negatively charged oligonucleotide backbone to a negatively charged silica membrane inside the spin column under high salt conditions, followed by one or more washing steps and elution of the oligonucleotides under low salt conditions (e.g., using DNA Clean and Concentrator-25 kit from Zymo Research according to the manufacturer's protocol).

Optionally, a Step (4*) allows for an error-prone PCR in order to induce mutations, allowing an aptamer evolution. For details of preferred error-prone variants see below.

*Process Variant (bypass of bioactivity loop): If the bioactivity screening loop is to be bypassed in order to directly continue with the next AGE-SELEX round, the purified sample is directly subjected to the T7 Polymerase in vitro transcription step (refer to section H. in the Example).*

After Step (4) or as part of it, the resulting nucleotides in the oligonucleotide mixture obtained are preferably quantified by an appropriate method, especially by digital droplet PCR (ddPCR), preferably with samples comprising a promoter, e.g. the T7 promotor sequence. First, preferably a dilution series in a dilution buffer (e.g. 10 mM Tris HCl, pH 8.5, 0.1 mM EDTA) is prepared (x-fold dilution, with, for example, x = 5, 25, 100, 400, 1600 and 6400), in order to find an appropriate dilution with the dynamic range of the ddPCR of preferably about 1 to about 2'200 copies/μl (e.g., according to the 26K 24-well Qiagen protocol). If the cycle number of the preceding RT-PCR is changed, the dilution series is preferably adjusted accordingly. The PCR samples are then prepared using a PCR kit, such as the QIAcuity EG PCR Kit from Qiagen, to an appropriate volume, e.g., a reaction volume of 10 to 100 μl, preferably e.g. 40 μl, using well matrices with a large number of partitions, such as the 26k 24-well (with 26000 partitions for one reaction, one partition corresponding to a volume of 0.91 $\mu$l), using customary cycling conditions, e.g. those provided by the manufacturer. Based on the Poisson equation (see below), the copy number is calculated from the fraction of modified or unmodified DNA positive partitions. Values lying outside the dynamic range mentioned above are not used for further calculations. If multiple values lie within the range, the mean value is used.

*Note: The DNA quantification step is performed to get a precise quantification of the library at this point. Besides the information about the concentration of the PCR product from the RT-PCR step, no material is subject to a subsequent AGE-SELEX step.*

Based on the result, the amplified oligonucleotides from the RT-PCR step are then used to prepare a dilution with a statistical number of copies per ml, e.g., with y = 1000 copies/ml (also named $Ld_y$ sample hereinafter, with y being the number of copies/ml).

The resulting (modified or unmodified DNA) nucleic acid mixtures are then subjected in a Step (5) (that is a very important step in the AGE-SELEX process as it allows to single out the sequences of aptamers in separate fractions) to a limiting dilution endpoint PCR comprising the amplification of (statistically) single molecular nucleotide fractions of sequences in order to obtain fractions with (statistically) only one or very few different sequences, e.g., on average about 0.5 to about 5, such as about 1.6, sequences.

For later assessment of the biological activity of single aptamer candidates, the $Ld_y$, e.g. $Ld_{1000}$, sample is subjected to an array of (preferably 3840) Limiting Dilution Endpoint PCRs (LDEP PCR) with a mean concentration of 0.5 to 5, e.g. 1.6 oligonucleotides per partition corresponding to 0.5 to 5, e.g. 1.6 $\mu$l of the $Ld_{1000}$ sample as a template.

*Note: A number of 3840 reactions (10 x 384 well plates) is considered as an appropriate basic value representing a compromise between assay complexity and sample size allowing a statistical representation of the aptamer pool. However, this number may be adapted based on sequencing results from the total aptamer pool.*

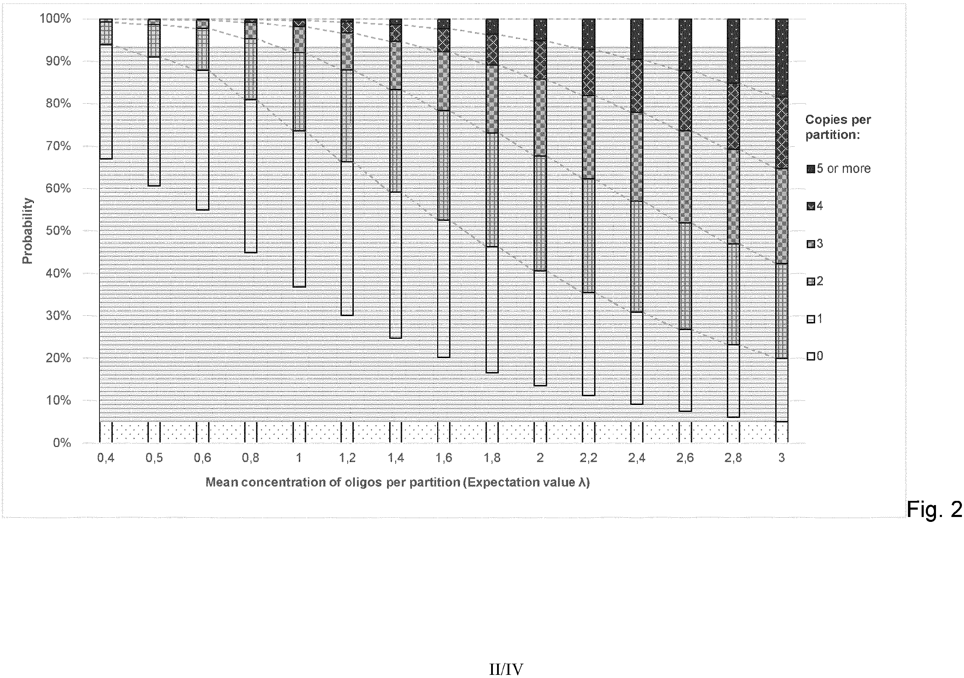

According to the Poisson distribution, a preferred mean concentration of 1.6 oligos per reaction corresponds to about 80 % of partitions with at least one oligonucleotide and is considered as a "low threshold filter" concentration to allow more oligonucleotides to be assessed in Step (7) within one bioactivity screening. Under such low-threshold filter conditions, it has to be considered that some oligonucleotides corresponding to inactive aptamers are able to pass the screening if they share one partition with an oligonucleotide corresponding to a bioactive aptamer. In later rounds however, remaining inactive aptamers can be sorted out if the mean concentration is lowered further to a "high threshold filter" condition below 0.6 oligonucleotides/partition. This allows to reduce the number of partitions with multiple oligonucleotides to less than 10 % (see Fig. 2).

The PCR reactions of the fractionated material thus obtainable are then performed in (e.g., bar-or matrix or otherwise coded) PCR plates using the number of plates corresponding to the required number of fractions, e.g. with ten such plates, e.g. with 384 wells per plate, using an appropriate partition volume, such as about 10 to about 50, e.g. about 30 $\mu$l. The PCR is performed with a suited DNA Polymerase (e.g., Q5 High-Fidelity Hot Strat DNA Polymerase from NEB) and the Primer-Pair LD + LD-1. The annealing and elongation conditions are set as appropriate, e.g., according to table 3 in the Example Section. To generate a maximum amount of DNA, a cycle number between 25 and 50, e.g., of 40, cycles is chosen. To monitor the reactions, preferably a DNA fluorescent stain is used, e.g., the fluorescent DNA stain EVA Green (Jena Bioscience) is used in the PCR buffer in a final concentration of about 0.05 to about 0.5 $\mu$M. This allows that during and after the PCR run, the sample fluorescence is monitored. Samples exceeding a set threshold (e.g., 30 % of maximum fluorescence) are recognized as positive fractions. The resulting ratio of positive partitions should be in proximity with the expected value according to the Poisson distribution (i.e., for example, for a mean concentration of 1.6 oligos per well, the amount of positive partitions is about 80 %). The mean calculation of oligonucleotides which are effectively present in the partitions $(\lambda_{is})$ can be calculated using Equation (A) in the Examples Section. This value can be compared with the theoretical value of $\lambda_{should}$ (e.g., $\lambda_{should}$ = 1.6 oligonucleotides per partition). If a systematic deviation from this target value should arise due to technical reasons, using Equation (B) represented below in the Example Section, a correction factor may be implemented to adjust the input volume of the $Ld_{1000}$ sample.

The resulting oligonucleotide fractions (amplicons) are then preferably subjected to a PCR purification step. Depending on the type of automated setup, all or only positive partitions ("cherry picking" operation) are purified, e.g., using affinity binding based on the charged backbone of the oligonucleotides to a negatively charged silica membrane coated onto paramagnetic beads under high salt conditions, subjecting the resulting bound material to one or more washing steps, and finally eluting the oligonucleotides under low salt conditions to achieve a desired volume, e.g., about 5 to about 100 or preferably about 15 to about 30 $\mu$l.

*Process Variant: As an alternative, the PCR product is isolated with suitable PCR purification filter plates (i.e., PCR Cleanup Filter Plates, Millipore or other suited plates) in order to process higher PCR reaction volumes.*

The resulting singled-out DNA sequences are then, in Step (6) (i), where RNA aptamers are desired, transcribed to yield the respective aptamers (as single stranded RNA or preferably as single stranded RNA derivatives, e.g. 2' modified RNA) of (essentially) single molecular nucleotide fractions of sequences (also named "single clone(s)" herein; for Example, the fractions are directly subjected to a Polymerase mediated (e.g., in case of (modified) RNA single strand (ss) molecules to be obtained from DNA, T7 Polymerase mediated) transcription step, using appropriate conditions known to the person skilled in the art, e.g., as described in the Examples Section. As an optional step, any remaining template DNA can be degraded by DNase treatment after the transcription step. A suited DNase (e.g., Turbo DNase, Invitrogen) is used, e.g., in an amount of 1 to 5 units to the reaction and incubated for about 30 minutes at 37 °C.

Where DNA aptamers are desired, Step (6) (ii) is employed where the desired DNA oligonucleotides are separated from the antisense-strand (which is discarded) to yield the single strand oligonucleotide DNA (or DNA derivative). In order to remove an anti-sense strand from the aptamer sense strand, it is preferably possible to either use a conventional PCR in Step (5) followed by a purification of the aptamer sense strand by the use of affinity tagged primer (e.g. biotin, using its binding to streptavidin on a solid support), or it is possible to make use of an asymmetrical PCR, or a combination of both.

After Step (6), in following Step (7), optionally the bioactivity (as defined above) of the obtained aptamers is determined, preferably after a buffer exchange. This bioactivity determination step adds an important second dimension to the known SELEX methods where only binding is determined, not biological activity, and thus is a central part of the present AGE-SELEX method, providing information that is important for selecting effective aptamers with the desired properties in binding and activity.

The bioactivity can be measured in many ways, depending on the target and the reaction elicited by its (especially natural) ligand (which, due to the biological activity it causes, can also be called an effector).

For examples of possible methods see above. Especially, molecules and/or reactions leading to a change in fluorescence or luminescence can be used.

In an important embodiment, the bioactivity measurement is made by using a quantitation reporter for gene expression in (especially eukaryotic) cells. Examples of such quantitation reporters include Green Fluorescent Protein (GFP), luciferase (Luc; e.g. *Renilla* luciferase) e.g. making use of a dual luciferase assay (transient) or a lentiviral dual-luciferase assay (stable). In order to achieve a fluorescence or an increase in fluorescence (or to diminish it, e.g., by induced expression of fluorescence quenching protein), where a ligand to be examined by binding to an, e.g., transmembrane protein accessible to the ligand causes a chain of events that leads to the activation or deactivation of the expression of a gene. Several membrane receptors are activated or inactivated by ligands, leading (often via signal molecules or signal cascades) to induced or reduced gene expression, for example, Epidermal Growth Factor Receptor, upon binding of the ligand EGF, dimerizes and its tyrosine kinase is activated. It is known that epidermal growth factor (EGF) stimulation of cells eventually leads to an activation of the serum response element (sre) promotor (e. g., Suzukawa, 2002). After the mentioned activation, subsequent signal transduction uses the MAPK/ERK, PI3-Kinase, small GTPases and other pathways (Lemmon, Cell 2020). Activation of the MAPK/ERK pathways results further downstream in phosphorylation and activation of serum response factor (SRF) and eventually of serum response element (SRE). Using reporter constructs like sre-GFP or sre-Luc containing repeated tandem SRE sequences (AGGATGTCCATATTAGGACATCT - SEQ ID NO: 1) which are commercially available or can be constructed conveniently (e.g., using SRE-Luciferase reporter construct, Agilent; sre-GFP lentiviral reporter construct, GenTarget LVP957R), gene expression of the fluorescent proteins under control of a sre can be used to detect EGF bioactivity. In a more general vein, gene expression modulation (activation or deactivation) under control of a promotor of a respective protein can be stimulated by activation or inhibition of the promoter. When the promoter controls expression of GFP or Luc increased or decreased fluorescence or luminescence is measurable, thus having a marker for bioactivity.

Appropriate preferred process variants for bioactivity measurement include the GFP based one described in Example 1 under I., or, for example, the following variants:

**Process Variant 1:** Wt-Caco-2 cells are seeded in white well, gelatin coated 384-well plates with 1000 cells per well in a total volume of 40 µl medium. At -25 - 30% confluency a transfection of pISRE-Luc by using lipofectamine is performed (cf. https://www.thermofisher.com/de/de/home/references/protocols/cell-culture/transfecti on-protocol/transfecting-plasmid-dna-into-caco-2-cells-using-lipofectamine-ltx-reagent.htm l#:~:text=Transfection%200f%20Caco%2D2%20Cells,-Use%20this%20procedure&text=Plate%204%20x%20104,on%20the%20da y%20of%20trans fection.&text=Incubate%20the%20cells%20at%2037,before%20assaying%20for%20transge ne%20expression). A dual-luciferase reporter assay system is used according to the instructions of the manufacturer (Promega; cf). 15 µl of each single aptamer PCR fractions is added to one well each and incubated on the cells for 6 h in starvation medium. Untreated wt-Caco-2 cells (negative) or wt-Caco-2 cells treated with 30 nM EGF for 10 min (positive) are used as controls. Firefly luciferase activity is measured in a luminometer and normalized on the basis of *Renilla* luciferase activity.

**Process Variant 2:** Lentiviral Dual-Luciferase Assay (stable) sre-GFP/Luc+-Caco-2 cells are generated as stable cell line through lentiviral transduction according to the manufactures protocol (e.g., Applied Biological Materials Inc. (abm), see https://www.abmgood.com/pub/media/catalog/customservice/custom-5-utr-reporter-vector/UTR-Reporters-Handbook-final.pdf) UTR GFP/Luc+-Caco-2 cells are seeded in white well, gelatin coated 384-well plates with 1000 cells per well in a total volume of 40 µl medium and grown to nearly confluency. 15 µl of each single aptamer fractions PCR is added to one well each and incubated on the cells for 6 h in starvation medium. Untreated UTR GFP/Luc[+]-Caco-2 cells (negative) or UTR GFP/Luc[+]-Caco-2 cells treated with 30 nM EGF for 10 min (positive) are used as controls. *P. pyralis* luciferase activity is measured in a luminometer and normalized on the basis of the constitutively expressed *R. reniformis* luciferase activity.

**Process Variant 3:** Promega Steady-Glo® Luciferase Assay System Wt-Caco-2 cells are seeded in white well, gelatin coated 384-well plates with 1000 cells per well in a total volume of 30 µl medium. At -25 - 30% confluency a transfection of pISRE-Luc by using lipofectamine is performed (e.g., cf. https://www.thermofisher.com/de/de/home/references/protocols/cell-culture/transfection-protocol/transfecting-plasmid-dna-into-caco-2-cells-using-lipofectamine-ltx-reaoent.html#:~:text=Transfection%200f%20Caco%2D2%20Cells,-Use%20this%20procedure&text=Plate%204%20x%20104,on%20the%20day%20of%20trans    fection.&text=Incubate%20the%20cells%20at%2037,before%20assaying%20for%20transge ne%20expression.)

After transfection the cells are cultured in 30 µl starvation medium for 12 hours. To start the stimulation, the medium is exchanged with a mixture of 15 µl single aptamer fraction and 15 µl starvation medium and incubated on the cells for 6 hours. Untreated wt-Caco-2 cells (negative) or wt-Caco-2 cells treated with 30 nM EGF for 10 min (positive) are used as controls.

Luciferase is measured with a commercially available kit (Promega, Steady-Glo® Luciferase Assay System, cat. Nr. E2550) according to the manufacture's manual. In brief: 30 µl kit reagent is added to each well of the 384-well plates containing 30 µl medium and mixed. After 5 minutes of incubation time, Firefly luciferase activity is measured in a luminometer and normalized on the basis of OD600.

**Process Variant 4:** 2-step rt-PCR (TurboCapture)

Wt-Caco-2 cells are seeded in white well, gelatin coated 384-well plates with 1000 cells per well in a total volume of 30 µl medium. Cells are grown to about 90 % confluency before starved in 30 µl starvation medium for 12 hours. To start the stimulation, the medium is exchanged with a mixture of 15 µl single aptamer fraction and 15 µl starvation medium and incubated on the cells for 6 hours. Untreated wt-Caco-2 cells (negative) or wt-Caco-2 cells treated with 30 nM EGF for 10 min (positive) are used as controls.

To isolate the respective mRNA from the cells, a commercially available kit is used (Qiagen, TurboCapture 384 mRNA Kit, cat. Nr. 72271). The procedure is performed according to the manufacture's manual. In brief, medium is removed and cells are washed gently with phosphate buffered saline (PBS). PBS is removed, lysis buffer is added and incubated for 5 min. Cell lysates are transferred onto a TurboCapture plate and incubated for 90 min on an orbital shaker (100 rpm). Eventually the plate is washed 3 times and cDNA synthesis is performed.

cDNA synthesis is performed with a commercially available kit and according to the manufacturer's protocol (Qiagen, Sensiscript RT Kit, cat. nr. 205213). In brief, the remaining buffer is removed from the TurboCapture plate and 20 µl of the prepared reverse-transcription reaction mix is added. The plate is incubated for 60 min at 37 °C. The plate is washed 3 times with 30 µl Tris-HCl (10 mM). Washing solution is discarded and a rtPCR is performed.

To perform the qPCR a commercially available kit is used (Qiagen, QuantiTect SYBR Green PCR Kit, cat. nr. 204145) according to the manufacturer's manual. 50 µl prepared rtPCR mix (1x QuantiTect SYBR Green PCR Master Mix, 0.3 µM Primers each, water) is added to each well. The fold-induction of c-JUN mRNA (primer sense: CGTGAAGTGACGGACTGTTC; anti-sense: GTAGCCATAAGGTCCGCTCT;) is measured as surrogate for EGFR induced signal transduction. The Plate is placed in a qPCR-cycler (Applied Biosystems ProFlex PCR System, cat. nr. 4484077) and following program is run:

| Step | Temperature [°C] | Time [s] |
|---|---|---|
| Initial heat activation | 95 | 900 |
| 3-step cycling (40 cycles) | | |
| 1. denaturation | 95 | 15 |
| 2. annealing | 55 | 30 |
| 3. elongation | 72 | 30 |
| Melting curve | 65 to 95 (ramp) | 15 |
| Hold | 8 | |

The first AGE-SELEX round is optionally and preferably performed without analyzing the bioactivity of the candidate pool. In, for example, Round 2 or 3, after narrowing down the candidate pool by two binding steps (Step (1)), a first bioactivity screen (Steps (4) to (7)) can then be performed. To streamline the AGE-SELEX process further, the bioactivity analysis is only performed in every n-th or in round n (n is a natural number lower than the full number of AGE-SELEX cycles employed), e.g., in AGE-SELEX Round 5 or Rounds 5, 7 and 10. Depending on target and library quality (as assessed within the AGE-SELEX process) the bioactivity analysis might also be performed in other or additional AGE-SELEX rounds. Preferably at least 2, 3, 4 or 5, more preferably 2 or 3 Rounds of the AGE-SELEX procedure include a bioactivity determination step.

For Steps (4) to (7) or instead of them, especially any one of the following protocols (A) or (B) may preferably be employed:

The library consists here of single stranded oligos with a variable region flanked by two constant regions necessary for primer binding. The variable region, for example, has a length of about 25 to about 60 bases. All or parts of the nucleic acids within the variable region consist of modified nucleic acids, e.g., 2'-modified RNA, and the employed Polymerase is selected so as to be able to accept the corresponding substrate. Note that these protocols can also be performed with unmodified DNA or RNA.

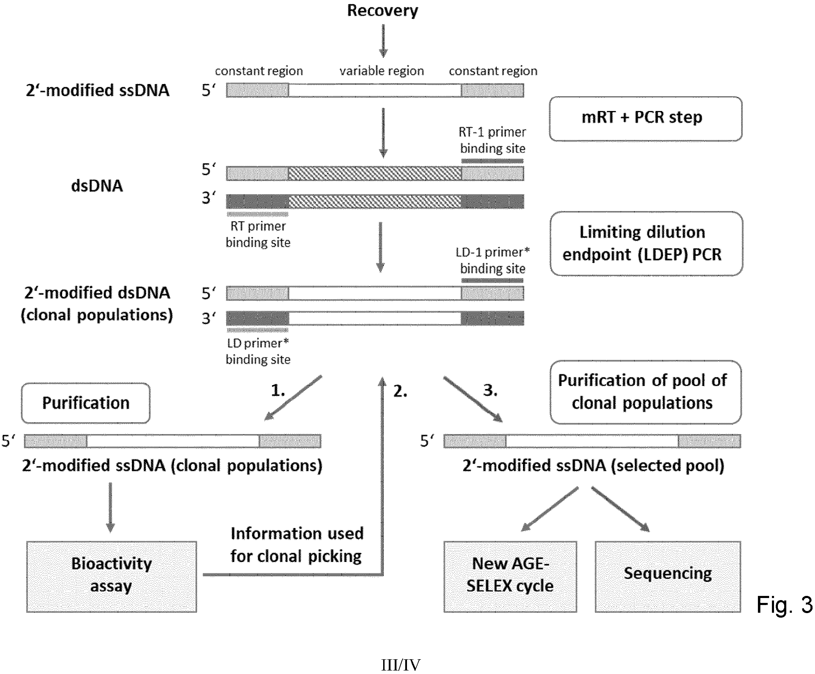

The protocols below describe two preferred alternative general ways how aptamers are amplified and purified. Depending on the actual protocol to be followed, the primer strategy is adapted accordingly. The main difference between both protocols is how the aptamer can be generated: either in the first protocol with a DNA Polymerase (protocol A for DNA based aptamers, see Fig. 3), or in the second protocol with a T7 RNA Polymerase protocol (protocol B for RNA based aptamers, see Figure (4)).

Protocol (A) (for DNA based aptamers):

Protocol A is essentially visualized and illustrated in Fig. 3

Fig. 3 illustrates a primer strategy and overview for protocol A without a transcription step for the generation of DNA based aptamers. White bars in the indicated variable region represent sequences including modified nucleic acids. The dashed bars in the indicated variable region represent sequences that consist of regular dsDNA. Sequences with flanking light grey bars in the indicated constant region represent the aptamer sense strand, those with dark grey bars represent the antisense strand. *Depending on the purification and PCR strategy, the primers LD and LD-1 bear an affinity tag (e.g., Biotin) and/or are designed to perform an asymmetric PCR.

Protocol A bears two DNA Polymerase amplification steps. The first one is a combined reverse transcription and PCR step. This refers to "mRT + PCR step" in Fig. 3, which corresponds to Step (4). This step is used to

- pre-amplify aptamers which may be lost otherwise,
- to precisely quantify the oligomers (via digital PCR) to improve the outcome of the limiting dilution endpoint PCR (LEDP) and to
- transcribe the modified nucleic acids back into conventional DNA.

The second DNA Polymerase step is used to amplify single clonal oligonucleotide populations. This refers to "Limiting dilution endpoint (LDEP) PCR" in Fig. 3, which corresponds to Step (5).

Each of the two branches 1. and 3. of "Purification" in Fig. 3 corresponds to Step (6) (ii) above, wherein branch 3. the Step (6) (ii) is part of the Step (9) and optionally Step (8).

The "Optional purification" (forming part of Step (6) (ii)) step may be conducted (here or in the more general method description) in order to remove the anti-sense strand from the DNA aptamer sense strand. This may be achieved either by a purification of the aptamer sense strand by the use of affinity tagged primer (e.g. Biotin) in the LDEP PCR step (Step (5)) or an asymmetrical LDEP PCR step (Step(5)) or a combination of both (e.g. by binding the biotinylated anti-sense strand to a solid support carrying streptavidin moieties, the sense-strand then remaining unbound, or by binding the biotinylated sense-strand to a biotin binding (e.g. streptavidin carrying) solid support material, washing out the anti-sense oligonucleotide and then eluting the sense-strand with biotin)).

The "Bioactivity Assay" corresponds to Step (7) above or below.

The purified and saved samples obtained in Step (5) or Step (6) (ii) corresponding to the aptamers with the desired bioactivity measured in Step (7) are then chosen,pooled and subjected to the next AGE-SELEX cycle or to a final sequencing step (for detailed description see Step (8) and Step (9) below).

Sequencing may optionally be conducted for partitions with singular oligonucleotide materials at the indicated stages. - END OF PROTOCOL (A)

Protocol B (for RNA based aptamers):

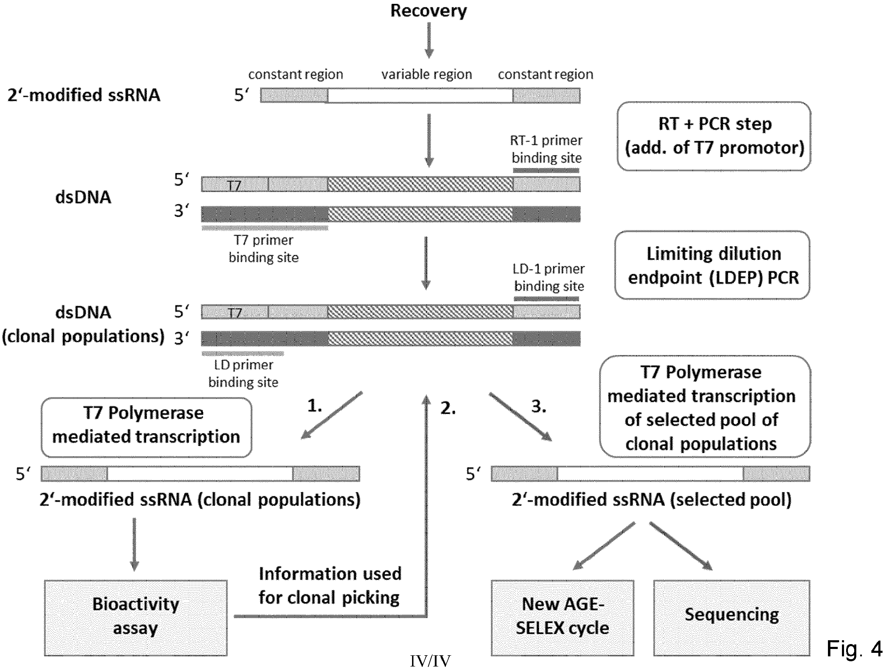

This protocol is essentially visualized and illustrated by Fig. 4:

Fig. 4 describes a primer strategy for general protocol B with transcription step for the generation of RNA based aptamers.

White bars in the indicated variable region represent sequences including modified nucleic acids and dashed bars. The dashed bars in the indicate variable regions that consist of regular DNA. Sequences with flanking light grey bars in the indicated constant region represent the aptamer sense strand, those with dark grey bars represent the antisense strand.

Protocol B bears two DNA Polymerase amplification steps and one T7 Polymerase transcription step and shares a high conceptual similarity with Protocol A. The main difference is that the clonal DNA populations have to undergo a final transcription prior to the bioactivity screening.

The "RT + PCR" step in Fig. 4 corresponds to Step (4).

The "Limiting dilution endpoint (LDEP) PCR" step corresponds to Step (5).

The single fraction dsDNA "dsDNA (clonal populations)" corresponds to the result of Step (5).

Each of the two branches 1. and 3. of "T7 mediated Polymerase transcription" in Fig. 4 corresponds to Step (6) (i) above, wherein in branch 3. the Step (6) (i) is part of the Step (9) and optionally Step (8)

Either, each fractional pool of (individualized) oligonucleotides (using an aliquot thereof) is subjected to "T7 polymerase mediated transcription" (corresponding to Step (6) (i) above) and can then be subjected to a bioactivity assay corresponding to Step (7) above or below. This provides information for picking bioactive "clones" which corresponds to branch 2. in Fig. 4 "information used for clonal picking".

The pools with bioactive oligonucleotides are then individually subjected to the "T7 Polymerase mediated transcription of selected pool of clonal populations" s tep and (either after a round (optional) or after the last AGE-SELEX round) sequenced, or if further AGE-SELEX rounds are to follow by a "New AGE-SELEX cycle". - END OF PROTOCOL (B).

The "Bioactivity Assay" corresponds to Step (7) above or below.

After any one of the general protocol Steps (4) to (7) (with optional omitting of Step (5) and Step (7)) or Protocols (A) or (B) the oligonucleotides/aptamers, in case of the inclusion of a bioactivity loop having shown bioactivity, are then used for the next step.

As an optional Step (8), a bioactivity screening feedback loop can be inserted (and is included in at least one AGE-SELEX round), closing the bioactivity loop, which is, however, omitted or preferably part of at least one of the AGE-SELEX rounds when the measured bioactivity (BA) of the candidate aptamers is significantly lower than the BA of the natural ligand, as it can allow to provide more specific and/or more affine aptamers in a higher amount, the aptamer fractions found to be active are subjected to an amplification, optionally including also an error-prone (mutagenic) PCR in order to induce mutations, thus allowing a kind of aptamer evolution, and then to the next AGE-SELEX round or another sequence of Steps (4) to (7); (Steps 4 to 8 thus forming an internal sub-round of the AGE-SELEX process, also called bioactivity feedback loop herein).

A preferred estimated trigger to perform a bioactivity feedback loop is: The average BA of 10 % most bioactive candidates is lower than $BA^{natural\ Ligand} * 0.9$.

To determine the relative bioactivity ($BA^{relative}$) of the bioactive candidates, preferably the following formula is applied:

$$BA(relative) = \frac{\dfrac{\text{Signal Intensity (bioactive candidate)}}{baseline\ read}}{\dfrac{\text{Signal Intensity (natural ligand)}}{baseline\ read}} * 100$$

Signal Intensity represents the quantified numeric value of the measured signal in the respective bioactivity assay, e.g., cell count, fluorescence, luminescence or absorption values. Baseline read represents an intrinsic assay control measurement, which depends on the bioactivity assay performed but whose signal is independent from the measured bioactivity signal; for example, in fluorescence based bioactivity assays baseline read is optical density of the sample measured at a wavelength of 600 nm (OD600).

To perform the bioactivity feedback loop, bioactive sequences are preferably selected by their measured BA. The cut off for the selection should be the 80 % most active sequences, but this may be varied in accordance with the AGE-SELEX round the step is performed in and also the measured relative bioactivity of the library overall. The corresponding dsDNA sequences for the selected aptamer saved in the Endpoint Limiting Dilution step are pooled and can be submitted to a mutagenesis PCR (error-prone PCR).

The error-prone PCR protocol (Step (4*)) is a special PCR protocol known to the person skilled in the art, designed to alter and enhance the natural error rate of the used polymerase. Using this step, aptamer candidates already identified with preferred binding and activity characteristics towards the target of interest can be further evolved in both of these characteristics by using their corresponding dsDNA as a template to derive a mutated aptamer sub-library potentially bearing improved aptamer candidates.

An increase in the error rate during a PCR step can be achieved, for example, by changing the buffer composition of the DNA polymerase, especially by using higher amounts of $MgCl_2$ or $MnCl_2$, or by the usage of a mutated DNA polymerase known to have a high intrinsic error rate.

After generating the mutant library, the resulting aptamers are subjected either directly to the AGE-SELEX binding Step (1) or recycled to another bioactivity sub-loop of AGE-SELEX starting with Step (5).

Directly after the first or after the last Step (7) or, where the recycling is bypassed, after the error-prone PCR, the selected aptamers are pooled in a Step (9) and the resulting pool is or the pools are then recycled into another AGE-SELEX round, including the sequence of Steps (1) to (7), with or without Steps (5) and (7), depending on the round, (and optionally (8)) and then Step (9).

Alternatively, in a Step (1), followed by Steps (2) to (7) (with or without Steps (5) and (7), depending on the round) and optionally (9), the pooled aptamers are incubated with cells presenting the target of interest or cells with a modified target of interest, e.g., a truncated version of a target (e.g., splice variants of the target, domain-deletion mutants of the target, a ligand-binding-interface mutated variant that still binds the ligand, an enzymatic inactive variant of the target (e.g., point mutated catalytic center), non-dimerizing versions of the target or chemically or enzymatically modified variants of the target (e.g. removal of glycosylation or PEGylation)), respectively. This helps to identify heteromultivalent aptamers or aptamers that are able to bind different (e.g. splice) variants of structural forms of a target. To that purpose, if desired, the binding target in subsequent AGE-SELEX cycles after round 1 can be altered. The alternation preferably follows a fixed sequence, depending on how many different target variants need to be included. E. g. for 3 targets (A, B, C) it would be like shown in the following table:

| Round | 1 | 2 | 3 | 4 | 5 | 6 | 7 | 8 | 9 | 10 |
|---|---|---|---|---|---|---|---|---|---|---|
| Target | A | B | C | A | B | C | A | B | C | A |

In a specific variant, where the AGE-SELEX method step is based on cells in cell culture plates, the AGE-SELEX cycle is performed as described above, but the cell culture plates are varied. To increase selection pressure, the plate size of the negative cells used in optional Step (3*) is increased, for example, from 35 to 150 mm, while the plate size of the wild-type cells is decreased, e.g., from 150 to 100 mm, and the time of washing is increased, e.g., from 15 to 30 min by Round 6. All cell plates are coated with the same carrier material, e.g., 0,1% gelatin.

Cell plate dish size (mm)

| Round | 1 | 2 | 3 | 4 | 5 | 6 | 7 | | 8 | 9 | 10 |
|---|---|---|---|---|---|---|---|---|---|---|---|
| Positive Selection (Step (1)) | 150 | 150 | 150 | 150 | 100 | 100 | 100 | | 100 | 100 | 100 |
| Negative Selection (Steps (1*) / (3*)) | - | 35 | 35 | 60 | 60 | 60 | 100 | | 100 | 150 | 150 |

An embodiment of the invention relates to the use of an aptamer found with the method according to the invention as a diagnostic, therapeutic or for research purposes, especially where the aptamer is used to modulate the activity of a growth factor receptor, e.g., that of EGFR; or to an aptamer for said use.

An invention embodiment also relates to a method of therapeutic treatment of a warm-blooded animal, especially a human, in need of such treatment, especially of a disorder caused by malfunction of a growth factor receptor, such as EGFR, comprising administering an aptamer found with the method according to the invention in a therapeutically effective amount to said warm-blooded animal, especially human.

An embodiment of the invention also relates to an aptamer found by the method of the invention that is active in modulating a target of interest, e.g., mimicking the binding of a ligand to a receptor, e.g., the binding of EGF to EGFR.

An invention embodiment also relates to the use of an aptamer according to the invention for the preparation of a diagnostic or medicament for diagnosing or treating a disorder that is dependent on a target of interest, e.g., a growth factor receptor, such as EGFR.

An invention embodiment also relates to the manufacture of an aptamer in larger scale or large scale, comprising the chemical and/or enzymatic (especially polymerase catalyzed) manufacture of said aptamer based on the sequence information from the (especially according to the bioactivity determination) most active and/or most specific or otherwise most interesting aptamer identified by the methods according to the invention.

The invention also relates to the embodiments defined by the claims, which are to be regarded as incorporated here.

Short description of the Figures:

Fig. 1: This figure is showing a representation of a complete round of an AGE-SELEX process including obligatory and optional steps according to the invention,

Fig. 2: Poisson distribution of oligonucleotides within reaction wells. Depending on the mean concentration of oligonucleotides, the number of partitions with 0, 1, 2, 3, 4 or more oligonucleotides changes accordingly.

Fig. 3: Primer strategy and overview for protocol A without a transcription step for the generation of DNA based aptamers. White bars in the indicated variable region represent sequences including modified nucleic acids. The dashed bars in the indicated variable region represent sequences that consist of regular dsDNA. Sequences with flanking light grey bars in the indicated constant region represent the aptamer sense strand, those with dark grey bars represent the antisense strand.

Fig. 4: Primer strategy for general protocol B with transcription step for the generation of RNA based aptamers. White bars in the indicated variable region represent sequences including modified nucleic acids and dashed bars. The dashed bars in the indicate variable regions that consist of regular DNA. Sequences with flanking light grey bars in the indicated constant region represent the aptamer sense strand, those with dark grey bars represent the antisense strand.

Examples Section

The following examples illustrate the invention without limiting the scope thereof:

**Example 1: AGE-SELEX Method applied to EGF receptor**

**(I) Materials**

Cell Lines and Cell Culture

Caco-2 cells (ATCC HRB-37) (also called wt for wild type) are cultured in high glucose DMEM (Corning; 10 0130CV), with sodium pyruvate and L-glutamine, 10 % heat inactivated fetal bovine serum (FBS) (Gibco, 26140079), 1X MEM non-essential amino acid solution (Sigma, M7145), and 1 % penicillin-streptomycin (Gibco, P0781). FBS is heat-inactivated by heating at 56 °C for 30 min followed by cooling on ice. The cells are cultured on 0.1 % gelatin (Sigma, G1890) in culture plates (= culture dishes).
Cell Starvation medium is the respective cell line medium without the addition of FBS.

Generation of EGFR-null Caco-2 cells

EGFR-null Caco-2 cells are generated with the EGFR Human Gene Knockout Kit (CRISPR) (Origene, KN214877; cf. https://cdn.origene.com/assets/documents/crispr-cas9/crispr%20knockout%20kit.pdf). The knockout is performed according to the manual of the manufacturer. In brief, wt-Caco-2 cells are seeded in a 6-well plate and grown to 70 % confluency. A Turbofectamin transfection of control (scrambled sequence), gRNA1 and gRNA2 constructs is performed. The transfected cells are grown for 48 h and splitted 1:10. For the next 18 days, cells are grown for 3 days and then split 1:10 again. The final passage is treated with puromycin to apply a selection pressure. Single cell colonies of puromycin resistant cells are picked and a genomic PCR is performed. The successful integration is confirmed by sequencing the amplified genomic fragment.

Confirmation of cell integrity

Flow cytometric assays using antibodies against cellular surface markers (e. g. CD10, CD13 CD26, CD14, CD35 and CD63, CD18, CD61 as described by Rodriguez-Juan C et al., Tissue Cell. 2001;33(6):570-579. doi:10.1054/tice.2001.0212) and the target are performed on a regular basis to confirm cell line integrity and maintain high quality.

Natural Ligand

Human EGF (EGF) (Sigma, E9644) is used as the natural ligand during elution steps.

Lentivirus

For gene expression assays, the sre-GFP lentiviral reporter construct (GenTarget LVP957-R) is used.

Buffers

The following buffers are used where mentioned:

| Buffer | Buffer composition |
|---|---|
| Wash buffer | $1 \times$ DPBS containing 4.5 g.l$^{-1}$ glucose and 2 mM $MgCl_2$ |
| Binding Buffer | $1 \times$ DPBS containing 4.5 g.l$^{-1}$ glucose and 2 mM $MgCl_2$ |
| Cell Suspension Buffer | $1 \times$ DPBS containing 4.5 g.l$^{-1}$ glucose, 2 mM $MgCl_2$ and 2 g.l$^{-1}$ HSA |
| Cell Starvation Buffer | $1 \times$ DPBS containing 4.5 g.l$^{-1}$ glucose and 2 mM $MgCl_2$ |
| Ligand Buffer | $1 \times$ DPBS containing 4.5 g.l$^{-1}$ glucose and 2 mM $MgCl_2$ |
| TE buffer | 10 mM Tris-HCl containing 1 mM EDTA•Na$_2$, pH 8.0 |
| DPBS = Dulbecco's phosphate-buffered saline | |

Oligonucleotides and Library

Oligonucleotides are purchased as dried material from Integrated DNA Technologies Inc., resuspended in 1 X TE buffer to a concentration of 100 $\mu$M (total oligonucleotide concentration) and aliquoted. Aliquots are stored in the freezer at -20 °C. In brief, the following primers adapted from Burmeister et al., 2005 are employed:

| Sequence Name | Sequence (5' → 3') |
|---|---|
| T7 | CGTAATACGACTCACTATAGGGATAATGGAACGTTCTCG (T7 region underlined) SEQ ID NO: 2 |
| RT-1 | CATCGATGCTAGTCGTAACGATCC SEQ ID NO: 3 |
| LD | CGTAATACGACTCACTATAGGGATAATGG SEQ ID NO: 4 |
| LD-1 | CATCGATGCTAGTCGTAACGATCC SEQ ID NO: 5 |
| LIB | GGGAUAAUGGAACGUUCUCG-(N$^{OMe}$)$_{50}$-GGAUCGUUACGACUAGCAUCGAUG SEQ ID NO: 6 |

The sequence library (sequence name: LIB) is designed as follows: The variable region of 50 2'-O-methyl-modified ribonucleotides ((N$^{OMe}$)$_{50}$) is flanked by one constant region each at the 5' and 3' terminus.

Polymerase

The following polymerases are used where mentioned:

| Name | Description | Reference |
|---|---|---|
| QuantiNova | DNA polymerase (present in QIAcuity EG PCR kit, Qiagen) for digital PCR step | Manufacturer's instruction for use |
| Q5 High-Fidelity | High-Fidelity DNA polymerase (present in Q5 Hot Start High-Fidelity 2X Master Mix, NEB) for the PCR amplification following the reverse transcription step and for the limiting dilution endpoint PCR (LDEP PCR). | Manufacturer's instruction for use |

(continued)

| Name | Description | Reference |
|------|-------------|-----------|
| T7 polymerase | T7 polymerase mutant RGVG M6 for the T7 polymerase-mediated transcription. | Meyer *et al*., 2015 Sugiyama *et al*., 2009 |
| Taq polymerase | Taq polymerase (Roche) for the error-prone PCR | Manufacturer's instruction for use |
| Mutazyme II | Mutazyme II polymerase (present in GeneMorph II Random Mutagenesis Kit, Agilent Technologies) for the error-prone PCR. | Manufacturer's instruction for use |
| Reverse Transcriptase SuperScript IV | Reverse transcriptase (present in First-Strand Synthesis System Kit, Invitrogen) for the reverse transcription step | Manufacturer's instruction for use |

**(II) Method**

Library Design and Primer Strategy:

The library consists of single stranded RNA oligonucleotides with a variable region flanked by two constant regions necessary for primer binding. The variable region has a length of 25 to 60 bases. All or parts of the RNA within the variable region consist of modified nucleic acids, e.g., 2'-modified RNA, provided the employed Polymerases is able to accept the corresponding substrate. Note that this protocol can also be performed with unmodified RNA.

In this example, a protocol comprising a T7 RNA Polymerase step according to Protocol B (Fig. 4) mentioned above (also named "main" protocol) is used.

A. Library preparation and target binding (special variant of Step (1) of the general process)

To perform the initial binding, twenty nanomoles of an oligonucleotide library is prepared. The oligonucleotide library is diluted in binding buffer and heated at 95 °C for 10 min, followed by chilling on ice for 10 min. Wt-Caco-2 cells are seeded on 0.1 % gelatin in a 150 mm cell culture dish and grown to confluency. The initial binding is performed by incubating the library with the wt-Caco-2 cells for 20 min at 37 °C.

B. Elution of bound oligonucleotides (special variant of a first part of Step (2) of the general process)

After washing 3 times with wash buffer, bound oligonucleotides are eluted by incubating the cells with 50 ng/ml EGF in 20 ml ligand buffer for 20 min at 4 °C. The ligand buffer containing the unbound ligand thus set free and the eluted oligonucleotides is heated for 10 min at 95 °C to inactivate the unbound ligand.

C. Negative Selection (special variant of Step (3*) = (1*) of the general process)

Note: This step is skipped (by-passed) in round 1 of the AGE-SELEX selection process.

A negative selection step is performed in order to remove false positive candidate sequences. The candidate sequences recovered in B. are chilled on ice for 10 min. EGFR-null CaCo-2 cells are seeded on 0.1 % gelatin in a 100 mm cell culture dish and grown to confluency. The binding is performed by incubating the recovered candidate sequences with the EGFR-null Caco-2 cells (20 min; 37 °C). After the binding step is completed, the supernatant is gently removed. The cells are gently washed for one time with 10 ml ligand buffer. The supernatant is added to the 10 ml wash ligand buffer and heated for 10 min at 95 °C. The cells used for negative selection and corresponding bound oligonucleotides are discarded.

D. Recovery (special variant of Step (3) of the general process)

The inactivated ligand buffer solution containing the eluted oligonucleotides fraction (after the first AGE-SELEX cycle or after the last Negative Selection Round (C. above) in subsequent AGE-SELEX cycles) is centrifuged at 16.000 x g for 10 min at 4 °C, purified with magnetic silica beads (MagMAX Cell-Free Total Nucleic Acid Isolation Kit, Thermo Scientific) according to the manufactures instruction and eluted in a volume of 15 µl elution buffer (e.g., 10 mM Tris-HCl, pH 8.5, 0.1 mM EDTA).

**Note:** The protocol is scaled according to employed volume of ligand buffer and performed without Proteinase K digestion.

E. Combined Reverse Transcription and PCR (special variant of Step (4) of the general process)

The reverse transcription and the amplification of isolated aptamer candidate oligonucleotides are performed in a two-step reaction. First, a reverse transcription step is performed according to Burmeister *et al.,* 2005 with the Superscript IV Reverse Transcriptase (Invitrogen). Accordingly, the aptamer candidate pool is annealed to the RT-1 primer and reversely transcribed with the SuperScript IV First-Strand Synthesis System (Invitrogen) according to the manufacturer's instruction in a total volume of 100 $\mu$l.

Hereafter, the reaction mixture is subjected to a buffer exchange with Sephadex G-25 spin columns according to the manufacturer's protocol (MicroSpin G-25 Columns,Cytiva). The underlying principle is a size exclusion chromatography process allowing to pass large molecules (like oligonucleotides) through the column and while retaining small molecules like salts and nucleotides.

The eluate is directly subjected to the following PCR step. The PCR is performed with DNA Polymerase (Q5 High-Fidelity Hot Start DNA Polymerase from NEB), the Primer-Pair T7 + RT-1 (0.5 $\mu$M each; Primer T7 contains the T7 Promotor flanking region), 1x concentrated buffer (Q5 reaction buffer from NEB containing 2 mM Mg$^{2+}$) in a total volume of 300 $\mu$l. The annealing and elongation conditions are set as follows:

*Table 1. Experimental conditions for LDEP PCR, according to the manufacturer's instruction for the Q5 Hot Start High-Fidelity 2X Master Mix from NEB.*

| Step | Temperature [°C] | Time [s] |
|---|---|---|
| Initial heat activation | 98 | 30 |
| 3-step cycling (15 cycles) | | |
| 1. denaturation | 98 | 10 |
| 2. annealing | 64 (cycle 1-2), 68 (cycle 3-15) | 20 |
| 3. elongation | 72 | 15 |
| Final extension | 40 | 120 |
| Hold | 8 | |

Hereafter, the PCR product is subjected to a spin column-based purification (DNA Clean and Concentrator-25 kit, Zymo Research, according to the manufacturer's protocol). In brief, the method is based on the high affinity of the negatively charged oligonucleotide backbone to a negatively charged silica membrane inside the spin column under high salt conditions. After one or multiple washing steps, the bound oligonucleotides are eluted under low salt conditions.

**Note:** Depending on the AGE-SELEX round (see L.) a variation of the combined reverse transcription and PCR step can be performed.

Process Variant -Error-prone PCR / Mutagenesis: (special variant of Step (4*) of the general process)

To force sample variability by mutagenesis, the PCR is also optionally performed as an error-prone PCR with the Mutazyme II polymerase (GeneMorph II Random Mutagenesis Kit, Agilent Technologies) according to the manufacturer's instruction. For more information on when the error-prone PCR is to be performed, refer to L. For the method using Mutazyme II polymerase, see above.

Process Variant - Bypass of bioactivity loop:

If the bioactivity screening loop is to be bypassed in order to directly continue with the next AGE-SELEX cycle, the purified sample is directly subjected to the T7 Polymerase in vitro transcription step (see H.). For more information on when the bioactivity loop is to be bypassed in the present example, refer to L.

F. Digital PCR (special variant of quantification in Step (4) of the general process)

To precisely quantify the amplified oligonucleotides, now bearing the T7 promoter sequence, they are subjected to a digital droplet PCR (ddPCR). First, the following dilution series is prepared (x-fold dilution by using dilution buffer (10 mM Tris-HCl, pH 8.5, 0.1 mM EDTA)): 5, 25, 100, 400, 1'600 and 6'400. This dilution series is necessary to find an optimal dilution within the dynamic range of the ddPCR of about 1 to 2.200 copies/$\mu$l (according to the 26K 24-well Qiagen protocol). If the cycle number from the foregoing RT-PCR is changed, the dilution series is adjusted accordingly.

The PCR samples are prepared with the QIAcuity EG PCR Kit (Qiagen) according to the manufacturers protocol with 8 $\mu$l of diluted sample, primers LD + LD-1 and a total reaction volume of 40 $\mu$l. The nanoplate 26k 24-well (Qiagen, 26,000 partitions for one reaction, one partition corresponds to a volume of 0.91 nl) is used. The cycling conditions are chosen as follows:

*Table 2. Experimental conditions for digital droplet PCR, according to the manufacturer's instruction for the QIAcuity EG PCR Kit from Qiagen.*

| Step | Temperature [°C] | Time [s] |
|---|---|---|
| Initial heat activation | 95 | 120 |
| 3-step cycling (40 cycles) | | |
|    1. denaturation | 95 | 15 |
|    2. annealing | 55 | 15 |
|    3. elongation | 72 | 15 |
| Cooling down | 40 | 300 |

Based on the Poisson equation (see Fig. 4), the copy number is calculated from the fraction of DNA positive partitions. Values lying outside of the dynamic range (see above) are not used for further calculations. If multiple values lie within the range, the mean value is used. Based on the result, the amplified oligonucleotides from the RT-PCR step are used to prepare a dilution with 1000 copies/ml (referred to as Ld1000 sample) which is used in G.

**Note:** This step is performed to get a precise quantification of the library at this point. Only the information about the concentration of the PCR product from the RT-PCR step is retrieved here.

G. Limiting Dilution Endpoint PCR (special variant of Step (5) of the general process)

To assess the biological activity of single aptamer candidates, the $Ld_{1000}$ sample is subjected to an array of 3840 Limiting Dilution Endpoint PCRs (LDEP PCR) with a mean concentration of 1.6 oligonucleotides per partition corresponding to 1.6 $\mu$l of the $Ld_{1000}$ sample as a template.

**Note:** A number of 3840 reactions (10 x 384 well plates) is considered as a basic value representing a compromise between assay complexity and sample size allowing a statistical representation of the aptamer pool. However, this number can be adapted based on sequencing results from the total aptamer pool.

According to the Poisson distribution (see Fig. 2), a mean concentration of 1.6 oligonucleotides (oligos) per reaction corresponds to about 80 % of partitions with at least one oligo and is considered as a "low threshold filter" concentration to allow more oligos to be assessed within one bioactivity screening. Under such low threshold filter conditions, it has to be considered that some oligonucleotides corresponding to inactive aptamers are able to pass the screening if they share one partition with an oligonucleotide corresponding to a bioactive aptamer. In later rounds however, remaining inactive oligonucleotides are sorted out if the mean concentration is lowered further to a "high threshold filter" condition below 0.6 oligonucleotides/partition. This reduces the amount of partitions with multiple oligonucleotides to less than 10 % (see Fig. 2).

The PCR reactions are performed in ten 384 well PCR plates with a partition volume of 30 $\mu$l. The PCR is performed with a DNA Polymerase (Q5 High-Fidelity Hot Start DNA Polymerase, NEB) and the Primer-Pair LD + LD-1. The annealing and elongation conditions and cycle numbers are set according to table 3. To monitor the reactions, the fluorescent DNA stain EVA Green (Jena Bioscience) is used in the PCR buffer (1x concentrated Q5 reaction buffer from NEB containing 2 mM $Mg^{2+}$) in a final concentration of 0.5 $\mu$M.

*Table 3. Experimental conditions for LDEP PCR, according to the manufacturer's instruction for the Q5 Hot Start High-Fidelity 2X Master Mix from NEB.*

| Step | Temperature [°C] | Time [s] |
|---|---|---|
| Initial heat activation | 98 | 30 |
| 3-step cycling (40 cycles) | | |
|    1. denaturation | 98 | 10 |
|    2. annealing | 66 | 15 |
|    3. elongation | 72 | 15 |
| Final extension | 40 | 120 |

(continued)

| Step | Temperature [°C] | Time [s] |
|---|---|---|
| Hold | 8 | |

During and after the PCR run, the sample fluorescence is monitored. Samples exceeding a set threshold (30 % of maximum fluorescence) are recognized as positive fractions. The resulting ratio of positive partitions is checked to be in proximity with the expected value according to the Poisson distribution (i.e., for a mean concentration of 1.6 oligos per well, the amount of positive partitions is about 80 %).

To calculate the mean concentration of oligonucleotides which are actually present in the partitions, the following Equation (A) is used:

$$Equation\ (A) \text{: } -ln\left(1 - \frac{amount\ of\ positive\ partitions}{amount\ of\ total\ partitions}\right) = mean\ concentration\ of\ oligonucleotide\ (\lambda_{is})$$

This value is compared with the theoretical desired value of $\lambda_{should}$ = 1.6 (oligonucleotides per partition). Where a systematic deviation from this target value arises due to technical reasons, a correction factor is implemented to adjust the input volume of the $Ld_{1000}$ sample based on the following Equation (B):

$$Equation\ (B) \text{: } corrected\ input\ volume\ Ld_{corr\ 1000} = \frac{\lambda_{should}}{\lambda_{is}} * input\ volume\ Ld_{1000}$$

The resulting amplicons are then subjected to the PCR purification step.

The rest of the samples, which is not used for the PCR purification step, is saved for the next AGE-SELEX round.

For the purification, 14 μl of the PCR reaction are subjected to a PCR purification (see AxyPrep Mag PCR Clean-up kit from Axygen) according to the manufacturer's manual. In brief, this method is based on affinity binding of the charged oligonucleotide backbone to a negatively charged silica membrane coated onto paramagnetic beads under high salt conditions. After multiple washing steps, the bound oligonucleotides are eluted under low salt conditions. The purification is performed by mixing the aliquot with 35 μl of a mixture of isopropanol (9.8 μl) and the magnetic bead suspension (25.2 μl) according to the manufacturer's protocol for DNA smaller than 100 bp and 384 well plates (magnet rack: 384-well Ring Magnet, Biotek). After purification, the sample is eluted in a volume of 15 to 30 μl and is directly subjected to the T7 Polymerase mediated transcription step.

H. T7 Polymerase mediated transcription (special variant of Step (6) (i) of the general process)

For the transcription reaction, the PCR products are transcribed with the T7 RNA Polymerase mutant RGVG M6 under the following reaction conditions: 200 mM HEPES pH 7.5, 5.5 mM MgCl$_2$, 2 mM spermidine, 0.5 mM 2'-O-methyl-NTP (each), 40 mM DTT, 0.01 % Triton, 10 % PEG8000, 1.5 mM MnCl$_2$, 10 U/ml yeast inorganic pyrophosphatase and 200 nM RGVG M6.

Depending on the AGE-SELEX round (see L.) and the subsequent step after the transcription, a distinct variation of the T7 Polymerase mediated transcription step is performed.

Process (I) - Preparing for Bioactivity measurement (which is corresponding to Step (7) in the general part): The final template concentration is estimated to lie in a range of 100 to 400 nM. The reactions are performed within suited 384 well plates with a reaction volume of 50 μl each. The reactions are incubated at 37 °C for up to 4 hours or overnight.

For the downstream bioactivity measurement, the samples are transferred into a 1000 Da MWCO centrifugation filter plate for a buffer exchange. After centrifugation at 3.000 to 4.000 x g, samples are filled up with the desired cell culture media and centrifuged again.

Optionally, this step is repeated once more. The concentrated aptamers with a volume of 15 μl are then subjected to the bioactivity measurement (see I.).

Process (II) - Preparing for Binding measurement: The template for the transcription is, depending on AGE-SELEX round (see L.), either:

- the partitions defined as active after the bioactivity measurement (see I.) and pooled and saved up in the LDEP PCR (see G.) or

- the partitions defined as active after the bioactivity measurement (see I.) and pooled and saved up in the LDEP PCR (see G.) which were subjected to an error-prone PCR (as described in E.) or
- the sample recovered from the RT-PCR step (see E.) - in case the bioactivity loop was bypassed (see L.)

In either case, the transcription is performed in a final template concentration of about 200 nM in a total volume of 300 μl at 37 °C for up to 4 hours or overnight. For the downstream binding measurement (see K.), the transcribed 2'-O-methyl-RNA oligonucleotides are purified with the Monarch RNA Cleanup Kit 50 μg (NEB, according to the manufacturer's protocol).

I. GFP based bioactivity assay (used in the present Example) (special variant of Step (7) of the general process)

To generate an EGF-sensible reporter cell lines, a sre-GFP lentiviral transduction is performed.
In preparation of the assay, sreGFP+-Caco-2 cells are generated by infecting wt-Caco-2 cells with sre-GFP lentivirus. The sreGFP+-Caco-2 cells are plated in gelatin coated 384-well black-wall cell culture plates (Greiner *M6936*; 1000 cells/well) and grown to confluency. Cells are starved in Cell starvation medium for 12 hours. 15 μl of one of the single oligonucleotide fractions is added to one well each and incubated on the cells for 6h in starvation medium. After replacing the medium with fresh starvation medium, the cells are subject to a multi-well reader to detect the GFP fluorescence signal. sreGFP+-Caco-2 cells treated with purification buffer (negative) or 30 nM EGF (positive) are used as controls.
The Plate Reader Setting for signal detection are as followed:

• chamber preheated to 37 °C
• For baseline read:

The transduced vector also contains a sequence for a constitutive expressed red fluorescent protein (RFP) which signal intensity can be used as baseline/normalization readout.
∘ RFP-read (EX: 545 nm / EM: 620 nm, normal reading speed, Gain: 50, bottom measurement, 10 data points per well).

• For GFP read:

∘ Fluorescence Endpoint (EX: 485 nm / EM: 510 nm, normal reading speed, Gain: 50, bottom measurement, 10 data points per well).

To normalize the reads, the signal is calculated as

$$s = \frac{GFP\ read}{baseline\ read} * 1000$$

Normalized signals exceeding a set threshold (35 % of maximum fluorescence) are recognized as positive fractions. The corresponding positive oligonucleotide fractions of this first round of selection are picked from the saved-up fractions (see G.) and pooled in binding buffer before being used in the next round of selection.

J. Transfer of bioactive candidates in another AGE-SELEX cycle (includes special variant of Step (9) of the general process)

Samples with a desired positive bioactivity are selected. From each of the selected samples, 5 μl from the saved rest (see G.) is taken and pooled, respectively. The pool is subjected to a spin column-based purification (DNA Clean and Concentrator-25 kit, Zymo Research, according to the manufacturer's protocol). The resulting purified pool is then subjected to the T7 Polymerase mediated transcription step used for the next AGE-SELEX cycle.

K. AGE-SELEX cycle (Round 2)

After determination of the candidate specific bioactivity the 80 % most bioactive aptamers are selected. The respective saved dsDNA sequences from Step I. are pooled, transcribed (see J.) and subjected to the subsequent AGE-SELEX cycle.
The biological active pool of aptamers derived from round 1 of the AGE-SELEX process is subjected to the next AGE-SELEX round. The aptamers are heated at 95 °C for 10 min, followed by chilling on ice for 10 min. The aptamers are incubated on the 100 mm positive incubation plate for 20 min at 4 °C. The following steps are according to round 1 of the cycle: 3x washing with 10 ml wash buffer and elution by incubating with 50 ng/ml EGF. The Elution is again heated for 10 min at 95 °C, followed by chilling on ice for 10 min.

After the Elution of the aptamers (positive selection step) a negative selection step is introduced in the AGE-SELEX cycle and performed in every subsequent round (see C.).

After performing the negative selection, the protocol is followed as described above, starting with Step (D.). (Recovery).

L. AGE-SELEX cycle variation overview for cycles 1 to 10

The AGE-SELEX rounds are performed as described above, but implementing the following protocol variations.

a. The cell culture plates are varied as shown in table 4 below. To increase selection pressure, the plate size of the negative cells is increased from 35 to 150 mm, the plate size of the wild-type cells is decreased from 150 to 100 mm, and the time of washing increased from 15 to 30 min by Round 6. All cell plates are coated with 0.1% gelatin.

*Table 4: Variations and performed steps in different AGE-SELEX rounds.*

| Round | 1 | 2 | 3 | 4 | 5 | 6 | 7 | 8 | 9 | 10 |
|---|---|---|---|---|---|---|---|---|---|---|
| Positive Selection (Cell plate size, mm) | 150 | 150 | 150 | 150 | 100 | 100 | 100 | 100 | 100 | 100 |
| Negative Selection (Cell plate size, mm) | no | 35 | 35 | 60 | 60 | 60 | 100 | 100 | 150 | 150 |
| Bioactivity | no | no | no | yes | no | no | yes | no | no | yes |
| Error-prone PCR | no | no | no | yes (after Step I) | yes | no | yes (after Step I) | no | no | no |

M. Sequencing

After round 10, a commercial high throughput sequence analysis (Illumina - Next Generation Sequencing) is performed to identify candidate sequences. The service provider performs blunt end ligation of sequencing primers on the submitted candidate pool. Using fluorescence signals, the integration of the different bases is measured, with the oligonucleotides being fixed via adapters to the surface of a solid support material. The resulting sequences serve as templates for the manufacture of the found aptamers in appropriate quantities

The AGE-SELEX cycle includes several optional steps which are only performed when specific criteria (named in the according optional steps, e.g., specific bioactivity level of the candidates) are met within one AGE-SELEX round. The optional steps provide the option to increase the quality of the results and evolve the candidate library in a desired way.

Optional - Bypass of bioactivity screen every n-th or in round n

The first AGE-SELEX round is performed without analyzing the bioactivity of the candidate pool. In AGE-SELEX round four, after narrowing down the candidate pool by four binding steps, the first bioactivity screen is performed. To streamline the AGE-SELEX process further, the bioactivity analysis is only performed in AGE-SELEX round 4, 7 and 10. Depending on target and library quality (as assessed within the AGE-SELEX process) the bioactivity analysis might also be performed in other or additional AGE-SELEX rounds.

Optional - Bioactivity screening feedback loop

The bioactivity screening feedback loop is only performed when the measured biologic activity (BA) of the candidates is significant lower the BA of the natural ligand. The general estimated trigger to perform a bioactivity feedback loop is: The average BA of 10 % most bioactive candidates < BA $^{\text{Ligand}}$ * 0,9.

To perform the bioactivity feedback loop, bioactive sequences are selected by their measured BA. The cut off for the selection should be the 80% most active sequences, but might vary due to the AGE-SELEX round the step is performed and also the measured relative bioactivity of the library overall. The corresponding dsDNA sequences for the selected aptamer saved in Step G. (Endpoint Limiting Dilution) are pooled and submitted to a mutagenesis PCR (error-prone PCR).

*Error-prone PCR*

The error-prone PCR protocol is a special PCR protocol designed to alter and enhance the natural error rate of the used polymerase. Depending on the Polymerase a distinct error pattern is favored.

By using the Taq polymerase the error is biased towards changes from AT to GC. Increasing $MgCl_2$ concentrations increase the error rate of the polymerase.

*default tag polymerase error-prone buffer:*

*10 mM Tris HCl (pH 8.3), 50 mM KCl, 7 mM $MgCl_2$, 0.3 $\mu$M of the primers T7 and RT-1 (each) and 0.05 UI$\mu$l Taq polymerase (Roche)*

*Default tag polymerase error-prone PCR protocol:*

| Step | Temperature [°C] | Time [s] |
|---|---|---|
| *Initial heat activation* | *94* | *30* |
| *3-step cycling (10 cycles)* | | |
| *1. denaturation* | *94* | *10* |
| *2. annealing* | *62 (cycle 1 and 2)* *67 (cycle 3 - 10)* | *30* |
| *3. elongation* | *72* | *60* |
| *Final extension* | *72* | *300* |
| *Hold* | *8* | |

By using a Mutazyme II polymerase (GeneMorph II Random Mutagenesis Kit, Agilent Technologies - performed according to manufacturer's manual) an error biased towards GC to AT can be introduced.

Depending on quality of the mutated library, it may be beneficial to combine multiple mutation steps by alternating between the used polymerases in successive generations, or by creating separate mutant libraries using both polymerases in a single generation.

After generating the mutant library, the result is subjected either subjected to the AGE-SELEX binding step or to the bioactivity sub-loop of AGE-SELEX.

References

- Adam J Meyer, Daniel J Garry, Bradley Hall, Michelle M Byrom, Hannah G McDonald, Xu Yang, Y Whitney Yin and Andrew D Ellington. Nucleic Acids Research, 2015, Vol. 43, No. 15, 7480-7488: Transcription yield of fully 2'-modified RNA can be increased by the addition of thermostabilizing mutations to T7 RNA polymerase mutants

- Paula E Burmeister, Scott D Lewis, Robert F Silva, Jeffrey R Preiss, Lillian R Horwitz, P Shannon Pendergrast, Thomas G McCauley, Jeffrey C Kurz, David M Epstein, Charles Wilson and Anthony D Keefe. Chemistry & Biology, 2005, Vol. 12, 25-33: Direct In Vitro Selection of a 2'-O-Methyl Aptamer to VEGF

- Sugiyama,A., Nishiya,Y. and Kawakami,B. U.S. Patent 7,507,567 B2, 2009: RNA Polymerase Mutants with Increased Thermostability.

- Lemmon MA, Schlessinger J. Cell signaling by receptor tyrosine kinases. Cell. 2010;

- Rodriguez-Juan C, Perez-Bias M, Valeri AP, et al. Cell surface phenotype and cytokine secretion in Caco-2 cell cultures: increased RANTES production and IL-2 transcription upon stimulation with IL-1beta. Tissue Cell. 2001;

- Soboleski MR, Oaks J, Halford WP. Green fluorescent protein is a quantitative reporter of gene expression in individual eukaryotic cells. FASEB J. 2005;

- Suzukawa K, Weber TJ, Colburn NH. AP-1, NF-kappa-B, and ERK activation thresholds for promotion of neoplastic transformation in the mouse epidermal JB6 model. Environ Health Perspect. 2002;

- Yordy, J., Muise-Helmericks, R. Signal transduction and the Ets family of transcription factors. Oncogene. 2000;

**Claims**

1. A method, named AGE-SELEX, comprising two or more selection rounds including the following steps:

   - In a first Step (1) - directly or after an additionally included preceding negative selection step as described below as Step (3*), with this preceding negative selection step here then referred to as Step (1*), using the resulting supernatant with an unbound oligonucleotide mixture - an oligonucleotide mixture (aptamer library), including DNA, RNA or XNA oligonucleotides comprising synthetic nucleotides preferably including terminal PCR primer sequences, is incubated with target carrying cells (preferably grown as monolayers) that display a target of interest or a modified target of interest; this is followed by
   - a Step (2) where the unbound oligonucleotides are removed by a washing step (and can be/are discarded);
   - in a subsequent Step (3) (a recovery step for the bound aptamers), the target carrying cells with the bound oligonucleotides are treated with an (especially natural) ligand to a target of interest, so that the specifically bound nucleotides are set free and then recovered from the resulting eluate of the target materials, especially cell cultures or cell suspensions;
   - optionally, in order to yet enhance the specificity of bound oligonucleotides to the target and to remove unspecifically bound oligonucleotides, a negative selection step is performed as an incubation Step (3*) with target negative cells (that is, cells different from the cells used in Step (2) not allowing for specific binding to the target of interest), especially cells inherently not exposing the target of interest, cells with altered targets, such as cells resulting from enzymatic inactivation or digestion of the target of interest or cells exposing a truncated or spliced target of interest, or in particular target knock-out cells follows, with subsequent removal of oligonucleotides that remain (unspecifically) bound on the cells,
   - while the supernatant resulting from Step (3) or optional Step (3*) with unbound oligonucleotides (candidate aptamers) is used in the next step, where
   - in a Step (4) forming part of the method according to the invention, the oligonucleotides are - if necessary (i) after Reverse Transcription (RT) into DNA starting from RNA or especially RNA analogues; or (ii) after (what we call here) modified Reverse Transcription (mRT) into DNA starting from DNA analogues (modified DNA) - amplified; where optionally Step (4) includes as Step (4*) an error-prone PCR in order to induce mutations, thus allowing a kind of aptamer evolution; and the concentration of the resulting oligonucleotide mixture is quantified before any subsequent Step (5), especially with digital droplet pcr;; and
   - the oligonucleotide mixture resulting from the amplification or the error-prone PCR is then if necessary, adjusted to a desired concentration and subsequently subjected in at least one of the AGE-SELEX rounds passed according to the method of the invention, preferably in two or more or all of the rounds, to a bioactivity determination, including a series of steps, together with Step 4 forming a bioactivity loop, as follows:

     ◦ in a Step (5) that is present in at least one of the rounds of the method but may be omitted in one or more rounds, the oligonucleotide mixture is subjected to a limiting dilution followed by amplification of (essentially) single molecular oligonucleotide fractions of sequences in order to obtain fractions with (essentially) only one, or very few different individual sequences; the fractions (partitions) with the resulting singled-out sequences are then partially saved while the rest, respectively, or where Step 5 is omitted (preferably aliquots of) the oligonucleotide mixtures of Step (4) or (4*), is or are subjected to
     ◦ a Step (6), where

       ▪ (i) either, where RNA aptamers are desired, the DNA oligonucleotides are transcribed to yield the respective RNA oligonucleotides as RNA or especially RNA derivatives, e.g., 2'-modified RNA, or
       ▪ (ii) where DNA aptamers are desired, the DNA oligonucleotides are either treated by separation of the desired sense-strand from the antisense-strand which is discarded, to yield the respective single strand sense oligonucleotides as DNA or DNA or Step (5) is performed as an asymmetrical PCR yielding to an excess of the desired sense-strand; or a combination of both;

◦ in a following Step (7) that is present in at least one of the rounds of the method but may be omitted in one or more other rounds - in such a case Step (5) is omitted as well - , the bioactivity of the oligonucleotides in different partitions obtained in Step (6) is determined (especially the modulation of a biochemical or biophysical reaction, e.g. causing an inhibition of a function (antagonist aptamers), an activation of a function (agonist aptamers) or other, e.g., complex, modulation) by individual testing, optionally followed by a reverse transcription and saving of aliquots of aptamer-sequences in the respective partitions for later sequencing;
◦ optionally, in a Step (8), in a bioactivity screening feedback loop, the oligonucleotide fractions found to be active are pooled and subjected to a further amplification Step (4), optionally including an error-prone PCR (as mentioned as Step (4*) above),
and with the resulting oligonucleotide mixtures of this Step (4) or (4*) either

- another Step (6) is performed followed by subjecting the resulting oligonucleotides to another sequence of Steps (1) / (1*) to (9), optionally including Step (8), said sequence of Steps constituting an AGE-SELEX round or
- another Step (6) is performed followed by subjecting the resulting oligonucleotides to another sequence of Steps (1) / (1*) to (9) where, however, Steps (5), (7) and (8) are omitted, thus leading to an oligonucleotide mixture pool (without individualized sequences), which bypasses the bioactivity determination, or
- another sequence of Steps (5) to (7), optionally including Step (8), is performed (additional bioactivity screening feedback loop);

◦ in a Step (9), an oligonucleotide pool is formed, where the oligonucleotide pool consists of either

- oligonucleotide fractions derived by a sequence of Steps (1) / (1*) to (7), optionally Step (8), where the oligonucleotides are selected based on their bioactivity performance in Step (7) and picked from the saved fractions (partitions) from Step (5), or
- oligonucleotide fractions resulting from an AGE-SELEX round where the bioactivity determination is skipped (bypassed) in that only the Steps (1) / (1*) to (6), omitting Step (5) (limiting dilution), are performed;

◦ the resulting pool is then either

- recycled into another AGE-SELEX round as described above (Steps (1) / (1*) to (9), optionally including Steps (5) and (7) and/or (8)) or
- into an AGE-SELEX round (Steps (1) / (1*) to (9), optionally including Step (5) and (7) and/or (8)), where the oligonucleotides are incubated (in a step analogous to Step (1) / (1*)) with cells comprising a modified target, e.g., a truncated or splice variant of a target or specific parts thereof that are thought to be or are of interest to be examined for being important for a biologic activity;

with the proviso that Steps (1), (2), (3), (4), (6) and (9) are obligatory in each round and both of Steps (5) and (7) are present in at least one round and/or preferably in the last round, and with the proviso that at the end of the last round, depending on the quality of the results of Step (7), Step (8) may be performed;
where in a final Step (10) an aptamer sequencing pool is generated including the oligonucleotide pool derived in Step (9) of the last AGE-SSELEX round and optionally oligonucleotide sequences saved in preceding AGE-SELEX rounds.

2. The AGE-SELEX method of claim 1, in which the cells are cells expressing a target of interest or if applied target negative cells, which in Step (1), Step (2) and Step (3) and if applied Step (1*) or (3*) are present as cell suspensions or especially as cell cultures, especially on cell culture plates.

3. The AGE-SELEX method according to any one of claims 1 or 2, wherein the number of rounds is from 2 to 20, preferably from 5 to 15, in particular from 8 to 12, such as 10 rounds before the found aptamer(s) is or are obtained.

4. The AGE-SELEX method according to any one of claims 1 to 3, wherein the bioactivity loop is used in the first or preferably the second round and subsequently in two to five, e.g., three, further rounds, e.g., in rounds (5), (7) and (10), and the number of rounds is preferably in the range from 3 to 15, especially from 8 to 12, such as 10.

5. The AGE-SELEX method according to any one of claims 1 to 4, wherein the bioactivity loop is bypassed in one or more rounds, e.g., in up to 8 rounds, such as in 6 rounds.

6. The AGE-SELEX method according to any one of claims 1 to 5, wherein the target of interest is one expressed on biological target materials preferably selected from cells, outer membranes of cells, preparations of the outer cell membrane or compartments in cells that communicate with the cell membrane, such as vesicles for internalization or secretion or externalization, preferably comprising Arc capsids, endocytosis vesicles or exocytosis vesicles or Golgi vesicles) carrying the target molecules, e.g., displayed on the outer cell membrane of a cell, moreover on membrane preparations of the cell membrane or compartment membranes in cells the biological activity of which is to be examined or used, especially activated or deactivated or otherwise modulated, e.g., for research, diagnostic or therapeutic purposes.

7. The AGE-SELEX method according to claim 6, wherein the target of interest is selected from the group consisting of bacterial pili, phage or virus binding protein, including glycoprotein, molecules expressed on the cells, such as ACE-2 or CD4 molecules; enzymes, e.g. lipase or protease secreting cells or especially cell membrane bound enzymes) ligand binding proteins, proteins involved in signal-transduction pathways, biomolecules involved in modulation of generation of second messengers, such as lipid messengers, nitric oxide, superoxide, hydrogen peroxide, carbon monoxide or hydrogen sulfides, biomolecules involved in modulation of generation or secretion of signal molecules in inflammation processes, intracellular signal or signal cascade and proteins and molecules associated therewith, e.g. protein kinases, especially serine/threonine, histidine or especially tyrosine kinases, extracellular receptors, e.g., G-protein-coupled receptors, Toll-like receptor, biomolecules involved in modulation of gene activation, such as activation or deactivation of any one or more of promotors, transcription, and translation, especially by ligand-mediated modulation of extracellular receptors, e.g. activation of sre-promoter leading to the expression of c-fos or when artificially coupled to reporter sequences, e.g., Green Fluorescent Protein, leading to their expression) (very preferred), intracellular receptors or the like; proteins showing reaction to growth factors (a preferred variant in all invention embodiments), e.g. EGF, based on binding of corresponding receptors present in the outer cell membrane, such as EGFR; biomolecules modulating cell-cell communication or interaction and molecules enabling it, such as hormones, tight junction proteins, modulation of proteins implied in adherens junctions or desmosomes, such as E-, N- and P-cadherins, desmoglein or desmocollin, gap junction proteins such as connexins; biomolecules involved in modulation of cell proliferation or cell division controlling proteins, apoptosis related proteins, such as TNFR1 or TNFR2, or Fas receptor or other caspase activators; molecules for the formation of cell walls; transmembrane proteins capable of modulation of electric membrane potential ,of ionic composition in the intracellular or especially extracellular medium, e.g., channels, such as leakage channels, ligand-gated channels, voltage-dependent channels, or ion pumps,
modulation of pH-regulation associated proteins, such as proton pumps, membrane bound transport proteins, e.g. ligand gated ion or proton channels, biomolecules involved in modulation of intracellular Calcium concentration; biomolecules involved in changes in metabolism e.g. inside the cell or modulation of transport proteins leading to release or consumption or exposure of organic molecules such as sugars, amino acids, lipids, peptides or nucleotides, biomolecules involved in modulation or expression or function of transmembrane proteins not yet mentioned; binding of antibodies or antigen binding receptor proteins specific for a membrane-bound component of cells e.g., MHC I or II or antigen receptors on leukocytes, e.g. lymphocytes); each of them either in isolated (especially outer) membranes of cells, compartments of cells (preferably as far as they interact with the medium outside cells, e.g. in endocytosis or exocytosis or Golgi vesicles), or preferably on intact (especially eukaryotic) cells in suspension or other cell culture, e.g. on dishes, and preferably exposing binding sites for the ligands uses on the outer cell membranes or in case of fungal or plant cells or bacteria also on their cell walls; especially an extracellular receptor (receptor proteins), such as a Growth factor receptor, in particular EGFR.

8. The AGE-SELEX method according to any one of claims 1 to 7 wherein the bioactivity feedback loop Step (8) is included once or twice, preferably also including the error-prone PCR step.

9. The AGE-SELEX method according to any one of claims 1 to 8, wherein the aptamer obtained in Step (10) is a DNA or DNA derivative or RNA or - especially 2'-modified - RNA derivative; where preferably the RNA derivative may be any derivative that avoids susceptibility to nucleases, including Spiegelmers, SOMAmers, locked nucleic acid (LNA), bridged nuclei acids (BNA), RNA with modified base pairs, such as 7-(2-thienyl) imidazo [4, 5-β] pyridine (Ds), 2-nitro-4-propynylpyrrole (Px), 5SICS-MMO2, SICS-NaM, 5SICS-DMO, isoG-5-Me-isoC, isoC-isoG, (enol)isoG-thymine or AF-Z, S-Pa base pairs, RNA comprising artificial nucleotides, e.g. 2-amino-8-(10-β-D-2-deoxyribofuranosyl)-imidazo[1,2-a]-1,3,5-triazin-4(8H)-one (P) or 6-amino-5-nitro-3-(10-β-D-20-deoxyribofur-anosyl)-2(1H)-pyridone (Z), RNA with modified bases, such as 5-methyluridine, pseudouridine, dihydrouridine or C5-ethynyl-deoxyuridine or its reaction product with azide (prefearably bound to a carrier material for synthesis of the aptamers), backbone modified RNAs, such as morpholino, peptide nuceic acid, phosphorothioate, boranophosphate variants, sugar modified nucleotide comprising RNAs, such as cyclohexenyl, altritol, arabinose, 2'-fluoroarabinose, 2'-fluoroarabino nucleic acid, preferably 2'-O-methyl ribonucleotide or especially 2'-fluoro ribonucleotide comprising RNA.

10. The AGE-SELEX method according to any one of claims 1 to 9, wherein the ligand preferably is a man-made/non-natural ligand or more preferably a natural ligand, e.g., an effector modulating a cell membrane receptor, especially EGF.

11. The AGE-SELEX method according to any one of claims 1 to 10 comprising at least one error-prone PCR step as part of Step (4) and/or at least one bioactivity screening feedback loop in Step (8).

12. A SELEX process including a step wherein an oligonucleotide mixture is subjected to a limiting dilution followed by amplification of essentially single molecular oligonucleotide fractions of sequences in order to obtain fractions with essentially only one, or very few different individual sequences; and wherein the fractions (partitions) with the resulting singled-out sequences can then be partially saved while the rest of the oligonucleotide is subjected to a next step in a customary SELEX cycle; wherein the step is preferably as described for Step (5) or for the combination of Step (5) and any one of Steps (6) (i) or (6) (ii) as defined in claim 1.

13. A bioactivity loop comprising Steps (4) to (7) and optionally Step (8) as defined in claim 1.

14. An AGE-SELEX method according to any one of the preceding claims 1 to 11, comprising a Protocol (A) as visualized in Fig. 3; or comprising a Protocol (B) as visualized in Fig. 4.

15. An aptamer showing a relative bioactivity that is at least 0.1 %, preferably at least 10%, more preferably at least 50 %, most preferably at least 90 % of the bioactivity of a natural ligand, said aptamer obtained by a method according to any one of the preceding claims and, if desired, modified and synthesized chemically in order to introduce DNA / RNA modifications not available with polymerase amplification, where in all cases mentioned the maximum relative bioactivity is preferably in the range between 100 % and 100 000 %, more preferably up to 100 %, of the bioactivity of the same concentration of the natural ligand, respectively.

Fig. 1

Fig. 2

Fig. 3

III/IV

Fig. 4

Europäisches
Patentamt

European
Patent Office

Office européen
des brevets

## EUROPEAN SEARCH REPORT

**Application Number**

**EP 21 18 1317**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/065404 A2 (AUGMANITY NANO LTD [IL]; AUMMUNE LTD [IL]) 2 April 2020 (2020-04-02) | 12,13 | INV. C12N15/11 |
| Y | * pages 10,11 * * pages 31,32,65; example 3 * | 1-11,14 | |
| Y | HASAN E. ZUMRUT ET AL: "Integrating Ligand-Receptor Interactions and In Vitro Evolution for Streamlined Discovery of Artificial Nucleic Acid Ligands", MOLECULAR THERAPY-NUCLEIC ACIDS, vol. 17, 1 September 2019 (2019-09-01), pages 150-163, XP055751056, US ISSN: 2162-2531, DOI: 10.1016/j.omtn.2019.05.015 * figure 1 * | 1-11,14 | |
| A | NOAM MAMET ET AL: "Discovery of tumoricidal DNA oligonucleotides by response-directed in vitro evolution", COMMUNICATIONS BIOLOGY, vol. 3, no. 1, 15 January 2020 (2020-01-15), XP055675369, DOI: 10.1038/s42003-020-0756-0 * the whole document * | 1-15 | |
| A | ZHONG YI ET AL: "Advances of aptamers screened by Cell-SELEX in selection procedure, cancer diagnostics and therapeutics", ANALYTICAL BIOCHEMISTRY, ACADEMIC PRESS, AMSTERDAM, NL, vol. 598, 19 February 2020 (2020-02-19), XP086150212, ISSN: 0003-2697, DOI: 10.1016/J.AB.2020.113620 [retrieved on 2020-02-19] * the whole document * | 1-15 | |

**TECHNICAL FIELDS SEARCHED (IPC)**

C12N

The present search report has been drawn up for all claims

| Place of search | Date of completion of the search | Examiner |
|---|---|---|
| The Hague | 13 December 2021 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 1317

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2021

| Patent document cited in search report | | Publication date | Patent family member(s) | | | Publication date |
|---|---|---|---|---|---|---|
| WO 2020065404 | A2 | 02-04-2020 | AU | 2019349873 | A1 | 06-05-2021 |
| | | | CA | 3114302 | A1 | 02-04-2020 |
| | | | CN | 113423833 | A | 21-09-2021 |
| | | | EP | 3856904 | A2 | 04-08-2021 |
| | | | KR | 20210096599 | A | 05-08-2021 |
| | | | WO | 2020065404 | A2 | 02-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7507567 B2, Sugiyama,A., Nishiya,Y. and Kawakami,B.

**Non-patent literature cited in the description**

- **Y. ZHANG et al.** *Molecules,* 2019, vol. 24, 941
- **MAN CHEN et al.** *Int. J. Mol. Sci,* 2016, vol. 17 (12), 2079
- **T. WANG et al.** *Biotechnology Advances,* 2019, vol. 37, 28-50
- **A. S. THOMPSON et al.** *Biochemistry,* 11 August 2020, vol. 59 (31), 2833-2841
- Crispr-Cas Methods. Springer Protocol Handbooks, 2020
- **RODRIGUEZ-JUAN C et al.** *Tissue Cell,* 2001, vol. 33 (6), 570-579
- **ADAM J MEYER ; DANIEL J GARRY ; BRADLEY HALL ; MICHELLE M BYROM ; HANNAH G MCDONALD ; XU YANG ; Y WHITNEY YIN ; ANDREW D ELLINGTON.** *Nucleic Acids Research,* 2015, vol. 43 (15), 7480-7488
- **PAULA E BURMEISTER ; SCOTT D LEWIS ; ROBERT F SILVA ; JEFFREY R PREISS ; LILLIAN R HORWITZ ; P SHANNON PENDERGRAST ; THOMAS G MCCAULEY ; JEFFREY C KURZ ; DAVID M EPSTEIN ; CHARLES WILSON.** *Chemistry & Biology,* 2005, vol. 12, 25-33
- **LEMMON MA ; SCHLESSINGER J.** Cell signaling by receptor tyrosine kinases. *Cell,* 2010
- **RODRIGUEZ-JUAN C ; PEREZ-BIAS M ; VALERI AP et al.** Cell surface phenotype and cytokine secretion in Caco-2 cell cultures: increased RANTES production and IL-2 transcription upon stimulation with IL-1beta. *Tissue Cell,* 2001
- **SOBOLESKI MR ; OAKS J ; HALFORD WP.** Green fluorescent protein is a quantitative reporter of gene expression in individual eukaryotic cells. *FASEB J,* 2005
- **SUZUKAWA K ; WEBER TJ ; COLBURN NH.** AP-1, NF-kappa-B, and ERK activation thresholds for promotion of neoplastic transformation in the mouse epidermal JB6 model. *Environ Health Perspect,* 2002
- **YORDY, J. ; MUISE-HELMERICKS.** R. Signal transduction and the Ets family of transcription factors. *Oncogene,* 2000